# EUROPEAN PATENT SPECIFICATION

(11) **EP 1 539 927 B1**
(45) Date of publication and mention of the grant of the patent: **18.03.2009**
(21) Application number: 03793503.8
(22) Date of filing: 08.09.2003
(51) Int. Cl.: C12N 1/20

(54) **PROBIOTIC BACTERIUM: LACTOBACILLUS FERMENTUM**
PROBIOTISCHES BAKTERIUM: LACTOBACILLUS FERMENTUM
BACTERIE PROBIOTIQUE: LACTOBACILLUS FERMENTUM

(30) Priority: 06.09.2002 AU 2002951270
(43) Date of publication of application: 15.06.2005
(73) Proprietor: Probiomics Limited, Eveleigh, NSW 1430 (AU)
(72) Inventor: CONWAY, Paricia, Lynne, La Perouse, NSW 2036 (AU)
(74) Representative: Van Someren, Petronella F. H. M.
(86) International application number: PCT/AU2003/001176
(87) International publication number: WO 2004/022727

(56) References cited:
- WO-A-01/97821
- WO-A1-02/34273
- CHARTERIS W P ET AL: "Development and application of an in vitro methodology to determine the transit tolerance of potentially probiotic Lactobacillus and Bifidobacterium species in the upper human gastrointestinal tract" JOURNAL OF APPLIED MICROBIOLOGY, vol. 84, no. 5, May 1998 (1998-05), pages 759-768, XP002337696 ISSN: 1364-5072
- PLANT, L. ET AL.: 'Association of lactobacillus spp. with Peyer's Patches in Mice' CLINICAL AND DIAGNOSTIC LABORATORY IMMUNOLOGY vol. 8, no. 2, 2001, pages 320 - 324, XP002990497
- HEINEMANN ET AL.: 'Purification and characterization of a surface-binding protein from lactobacillus fermentum RC-14 that inhibits adhesion of enterococcus faecalis 1131' FEMS MICROBIOLOGY LETTERS vol. 190, 2000, pages 177 - 180, XP000971010
- BLOMBERG ET AL.: 'Inhibition of adhesion of escherichia coli K88 to piglet Ilea1 mucus by lactobacillus spp.' APPLIED AND ENVIRONMENTAL MICROBIOLOGY vol. 59, no. 1, 1993, pages 34 - 39, XP001073684

## Description

### TECHNICAL FIELD

The present invention relates to variants of the bacterium *Lactobacillus fermentum,* formulations of the variant and components thereof, and their use in preventing and/or treating disease in mammals and promoting mammalian health.

### BACKGROUND

Any discussion of the prior art throughout the specification should in no way be considered as an admission that such prior art is widely known or forms part of common general knowledge in the field.

Indigenous bacteria play a major role in preventing certain bacterial and fungal diseases. They do this by a process of bacterial antagonism, preventing other microbes from establishing a presence in the body. Mechanisms involved in this activity include direct competition for nutrients, alterations in the acidity of an area making it hostile for other microbes, producing inhibitory metabolites and anti-microbial chemicals and by preventing other microbes from attaching to host surfaces.

Indigenous flora also influences the immune system. Animals reared in germ-free environments exhibit underdeveloped and relatively undifferentiated lymphoid tissues, low levels of immune proteins and a primary response to infection rather than a pronounced secondary response.

These microbes play an important role in the health of the host, by producing essential vitamins and nutrients required by the colonocytes, assisting with the degradation of complex nutrients, protecting the host from invasion by pathogens and stimulating the immune system. In addition, it has been proposed that the microbes in the bowel contribute to mineral absorption and lipid metabolism because of the lowered pH as a result of the short chain fatty acids produced by the microbes. These short chain fatty acids are regulators of colon physiology and play an important role in maintaining normal bowel function.

The upper gastrointestinal tract contains only bacteria swallowed with the saliva and food. As a result of the high acidity of gastric juices, few organisms, mostly lactobacilli, can be cultured from the normal stomach. The upper small intestine contains relatively sparse numbers af lactobacilli and enterococci. The flora of the gastrointestinal tract gradually changes until it becomes similar to that found in the colon, predominantly *Bacteroides, Bifidobacterium, Fusobacterium, Lactobacillus* and *Eubacterium.*

This normal flora of the body is a complex ecosystem, which is regulated by the diet, microbial interactions and host factors such as the motility of the gut and intestinal secretions. External factors such as stress, dietary changes and medications can affect the normal flora and alter the types of organisms present or their metabolism, If the balance is disrupted it can be detrimental to the host and can cause disease.

Sometimes, however, the balance is lost and invading pathogens are successful in penetrating the body's defences causing infections or triggering inappropriate immune responses. Similarly the balance between host and indigenous flora can be compromised leading to infection by normally dormant organisms, e.g. episodes of thrush (*Candida albicans* infection) or *Clostridium difficile* infection following the use of antibiotics.

Disappointingly, the "magic bullets" that antibiotics apparently offered have lost some of their impact. In the last 30 years, an ever-increasing problem of antibiotic resistance has become a major public health issue. Today, some antibiotics are all but useless against certain microbes, while some bacteria are resistant to almost all known antibiotic medications.

Further, evidence is accumulating that the indigenous microbes of the large bowel may be implicated in irritable bowel syndrome (IBS), a condition characterized by changes in bowel habit, disordered defaecation and distension. 8

A *Lactobacillus fermentum* KLD strain which preferentially binds to the follicle-associated epithelium of the Peyer's patches in the small intestine of the mouse is disclosed in Plant et al., Immunology 2001, 320-324.

Clearly alternative and perhaps complementary methods of treatment are required.

It is an object of the present invention to overcome or ameliorate at least one of the disadvantages of the prior art, or to provide a useful alternative.

WO 02/34273 discloses VRI 002 in inhibition of IL-4 secretion associated whit a T42 response.

### SUMMARY OF THE INVENTION

A new probiotic bacterial variant of *Lactobacillus fermentum* having surprising advantageous features over pre-existing strains of this bacterium as well as other probiotic microorganisms has been isolated. It is particularly useful in the prevention and/or treatment of gastrointestinal disorders due to its advantageous ability to colonise the gastrointestinal tract. Further, this variant has advantageous immunomodulatory effects, which occur both locally in the gut and at mucosal sites throughout the body via the common mucosal immune system. As a result of the commonality of the mucosal system the variant can also be used in the prevention and/or treatment of other disorders of mucosal surfaces and conditions resulting from mucosal surface disturbances or disturbances in the immune system or status of a subject.

According to a first aspect, there is provided a novel isolated *Lactobacillus fermentum* variant having the following characteristics:
(a) it is gram positive, facultative rod,
(b) it ferments ribose, galactose, glucose, fructose, mannose, maltose, lactose, melibiose, sucrose, trehalose, raffinose, L-arabinose and mannitol,
(c) it survives at pH 1.5 for up to 4 hours with a loss in growth of no more than log3,
(d) it propagates in the presence of 0.5% bile salts at 37°C resulting in an optical density increase of 0.5 to 0.8,
(e) it is stable when stored in gelatin capsules at 25°C,
(f) it adheres to Peyer's Patches at greater than log5 cfu per mg tissue, inhibiting pathogens both by a direct antagonistic effect and by triggering an immune modulation;
wherein said variant is VRI 003 (accession number NM02/31074).

According to a second aspect of the invention, there is provided a composition comprising a *Lactobacillus fermentum* variant according to the first aspect and a pharmaceutically acceptable carrier.

In a preferred embodiment, the composition comprises live cells of the *Lactobacillus fermentum* variant. However, it will be clear to the skilled addressee that the compostion may also contain dead cells of the Lactobacillus variant.

Preferably, the *Lactobacillus fermentum* is combined with other compounds such as prebiotics, non-digestible dietary components, dietary fibre or pharmaceutically active compounds. More preferably, the prebiotic comprises or consists of inulin, a resistant starch, an oligosaccharide, a gum or a beta-glucan. Even more preferably, the prebiotic is an unmodified high amylose maize starch or a beta-glucan.

The composition may be prepared as a tablet, capsule, powder, gel, paste, liquid formulation, dietary supplement or a food product and the like. Preferably, the composition is prepared in a tablet or capsule form

According to a third aspect, the invention provides the use of the *Lactobacillus fermentum* variant VRI 003 or a composition comprising the variant and a pharmaceutically active compound for the manufacture of a medicament for the prevention and/or treatment of a gastrointestinal disorder or a symptom thereof. The treatment comprises the step of administering a *Lactobacillus fermentum* variant according to the first aspect, or a composition according to the second aspect, to a subject in need of such treatment.

The terms "subject" and "individual" are used interchangeably in this specification and in the context of the present invention include within their scope any mammal which can develop, or already has, a gastrointestinal disorder and/or disorders of mucosal surfaces and/or conditions resulting from mucosal surface disturbances or disturbances in the immune system of whatever cause. The preferred subjects for administration of the treatment of the present invention are humans, domestic pets and farm animals.

Preferably, the gastrointestinal disorder is irritable bowel syndrome (IBS), inflammatory bowel disease, Crohn's disease and/or symptoms thereof, such as for example diarrhoea, bloating, flatulence, abdominal cramping, abdominal pain, or constipation. The gastrointestinal disorder may be caused by pathogenic organisms that may be bacterial, viral or protozoan. However, it may also be caused merely by colonisation of the gastrointestinal tract with inappropriate organisms or by inflammatory and/or autoimmune mechanisms.

Preferably, the disturbance or disorder is the result of colonisation of the subject's gastrointestinal tract by a pathogen. Preferably, the disturbance or disorder is the result of a high pathogen load as herein defined. More preferably the pathogen is a bacteria, virus or protozoa. Most preferably, the pathogen is *Salmonella, E. coli, Helicobacter, Vibrio, Pseudomonas, Clostridium,* bacteroides; or a virus such as Norwalks or rotavirus, or a protozoan such as *Cryptosporidium, Entamoeba, Giardia* and *Dientamoeba.*

In the context of the present invention, a "high pathogen load" may occur when a subject has (a) been subjected to attack by a pathogen in an amount not within the normal range of their everyday exposure; (b) been subjected to attack by a virulent pathogen; (c) been subjected to attack by a pathogen at a time when the subject's resistance is lowered, for example, at a time when the immune system is depleted and/or when the subject's other natural defence mechanisms are not functioning normally. The subject's resistance may be low due to, for example, stress or antibiotic treatment.

In a fourth aspect, the invention provides the use of *Lactobacillus fermentum* variant VRI 003 or a composition comprising the variant and a pharmaceutically active compound for the manufacture of a medicament for preventing and/or treating a disorder of a mucosal surface or a symptom thereof. The treatment comprises the step of administering a *Lactobacillus fermentum* variant according to the first aspect, or a composition according to the second aspect to a subject requiring such treatment.

Preferably, the disorder of the mucosal surface is eczema roseaca or atopic dermatitis.

In a fifth aspect, the invention provides the use of *Lactobacillus fermentum* variant VRI 003 or a composition comprising the variant and a pharmaceutically active compound for the manufacture of a medicament for stimulating IL-12 production in a subject. The treatment comprises the step of administering an effective amount of a *Lactobacillus fermentum* variant according to the first aspect, or a composition according to the second aspect, to a subject in need of such treatment.

In a sixth aspect, the invention provides the use of *Lactobacillus fermentum* variant VRI 003 or a composition comprising the variant and a pharmaceutically active compound for the manufacture of a medicament for up-regulating IFN-γ. The treatment comprises the step of administering an effective amount of a *Lactobacillus fermentum* variant according to the first aspect, or a composition according to the second aspect, to a subject in need of such treatment.

In a seventh aspect, the invention provides the use of *Lactobacillus fermentum* variant VRI 003 or a composition comprising the variant and a pharmaceutically active compound for the manufacture of a medicament for inducing a Th 1-type response in a subject. The treatment comprises the step of administering an effective amount of a *Lactobacillus fermentum* variant according to the first aspect, or a composition according to the second aspect, to a subject in need of such treatment.

In an eighth aspect, the invention provides the use of Lactobacillus fermentum variant VRI 003 or a composition comprising the variant and a pharmaceutically active compound for the manufacture of a medicament for inhibiting the growth of a pathogen. The treatment comprises the step of administering an effective amount of a *Lactobacillus fermentum* variant according to the first aspect, or a composition according to the second aspect, to a subject in need of such treatment.

In a ninth aspect, the invention provides the use of *Lactobacillus fermentum* variant VRI 003 or a composition comprising the variant and a pharmaceutically active compound for the manufacture of a medicament for modulating indigenous microbes of the gastrointestinal tract. The treatment comprises the step of administering an effective amount of a *Lactobacillus fermentum* variant according to the first aspect, or a composition according to the second aspect, to a subject in need of such treatment.

The required dosage amount will vary according to the severity of the condition to be treated, the cause of the condition, age of the subject and other standard clinical parameters which can be easily determined by routine procedures within the skill set of those skilled in the art.

The *Lactobacillus fermentum variant,* or the composition comprising said variant, may be administered by any known means but preferably it is administered orally.

The *Lactobacillus fermentum* variant, or the composition comprising said variant may be administered in conjunction with one or more other pharmaceutically active agents. The variant, or the composition comprising said variant, may be administered simultaneously (co-administered) with the other treatments or it may be administered sequentially in any order.

It is preferred that the *Lactobacillus fermentum* variant or a composition comprising it, be administered daily. It can be administered several times per day, or it may be administered infrequently (for example every second or third day), depending on the progress of treatment of the condition in question, its cause and severity. These parameters can also be easily determined by those skilled in the art.

In the context of the present invention, the term "stable" includes within its scope a loss in viability of up to 25% per six months' storage at 25 C.

Unless the context clearly requires otherwise, throughout the description and the claims, the words 'comprises', 'comprising', and the like are to be construed in an inclusive sense as opposed to an exclusive or exhaustive sense; that is to say, in the sense of "including, but not limited to".

### BRIEF DESCRIPTION OF THE FIGURES

Figure 1. Effect on different concentrations of bile salts on growth and viability of *L fermentum* VRI 003
**Figure 2**. The effect of low pH on viability of VRI 003 culture in vitro.
**Figure 3**. Adhesion of *Lactobacillus* spp to mouse Peyer's patches
**Figures 4** **and** **5**. A two-dimensional analysis of the cell wall proteins extracted from *L fermentum* VRI 003 cells compared to the cell walls of *L fermentum* LMG, previously shown not to adhere to the Peyer's patches, shows that the VRI 003 upregulates two cell wall proteins and downregulates the expression of a number of other cell wall proteins, (Figure 4) when compared to *L fermentum* LMG (Figure 5).
**Figure 6**. Effect of *L fermentum* VRI 003 stimulation on cytokine production by macrophages.
**Figure 7**. Enhancement of cytokine levels in the Peyer's patches following oral dosing of *L fermentum* VRI 003.
**Figure 8**. Effect of oral administration of *L fermentum* VRI 003 on cytokine levels in the spleen following oral dosing of *L fermentum* VRI 003.
**Figure 9**. Stability of *L fermentum* VRI 003 in Size 1 gelatin capsules combined with low water activity resistant starch and stored in foil-foil blister packs at 4C, 25C and 30C.
**Figure 10**. Changes in bacteria in faecal material from IBS subject #4 during an 8-week trial. Microbial profiles monitored during the baseline, placebo, washout and synbiotic treatment periods. Results expressed as colony forming units (cfu) per mL.
**Figure 11**. Changes in bacteria in faecal material from IBS subject #5 during an 8-week trial. Microbial profiles monitored during the baseline, placebo, washout and synbiotic treatment periods. Results expressed as colony forming units (cfu) per mL.
**Figure 12**. Changes in bacteria in faecal material from IBS subject #9 during an 8-week trial. Microbial profiles monitored during the baseline, placebo, washout and synbiotic treatment periods. Results expressed as colony forming units (cfu) per mL.
**Figure 13**. Changes in bacteria in faecal material from IBS subject #2 during an 8-week trial. Microbial profiles monitored during the baseline, placebo, washout and synbiotic treatment periods. Results expressed as colony forming units (cfu) per mL.
**Figure 14**. Changes in bacteria in faecal material from IBS subject #3 during an 8-week trial. Microbial profiles monitored during the baseline, placebo, washout and synbiotic treatment periods. Results expressed as colony forming units (cfu) per mL.
**Figure 15**. Changes in bacteria in faecal material from IBS subject #7 during an 8-week trial. Microbial profiles monitored during the baseline, placebo, washout and synbiotic treatment periods. Results expressed as colony forming units (cfu) per mL.
**Figure 16**. Changes in bacteria in faecal material from IBS subject #6 during an 8-week trial. Microbial profiles monitored during the baseline, placebo, washout and synbiotic treatment periods. Results expressed as colony forming units (cfu) per mL.

### DETAILED DESCRIPTION OF THE INVENTION

Although there have been various attempts to use probiotics in the prevention and treatment of many disorders, the evidence for the use of probiotics in the treatment of gastrointestinal disorders has been very variable. It has now been found that the *Lactobacillus fermentum* variant VRI003 having the following characteristics:
(a) it is a Gram positive, facultative rod,
(b) it ferments ribose, galactose, glucose, fructose, mannose, maltose, lactose, melibiose, sucrose, trehalose, raffinose, L-arabinose and mannitol,
(c) it survives at pH 1.5 for at least four hours with a loss in growth of no more than log 3,
(d) it propagates in the presence of 0.5% bile salts at 37°C resulting in an optical density increase of 0.5 to 0.8,
(e) it is stable with storage in gelatin capsules at 25°C,
(f) it adheres to Peyer's patches at greater than log5 cfu per mg tissue, inhibiting pathogens both by a direct antagonistic effect and by triggering an immune modulation;
is/are highly effective in the prevention and/or treatment of a gastrointestinal disorder and/or symptoms thereof. Preferably, the variant is *Lactobacillus fermentum* VRI 003. The present invention also provides the use of the variant or a composition comprising the variant for the preparation of a medicament for the prevention and/or treatment of a gastrointestinal disorder, the treatment comprising the administration of a *Lactobacillus fermentum* variant or a composition comprising the variant to a subject. The variant can be combined with other agents, for example, a prebiotic, a non-digestible dietary component, dietary fibre or a pharmaceutically active compound such as aspirin and statins.

The methods and compositions of the present invention have been developed for human and veterinary applications in the treatment of gastrointestinal disorders, but as a result of the commonality of the mucosal system, the treatments may be applied to other disorders of mucosal surfaces and conditions resulting from mucosal surface disturbances or disturbances in the immune system or status of a subject. Whether used for treatment of humans or domestic animals, the underlying principles are the same and advantageously the treatments of the present invention maybe used irrespective of the cause of the conditions described above.

Typically, the effective daily dosage is in the range of about 10⁸-10¹² bacteria and frequency of administration is once or twice daily. For long-term intake the amount may, for example, be below the above-mentioned range; and in other circumstances, it can be used at an amount above the range.

The *Lactobacillus fermentum* variant can be formulated by known means, using conventional pharmaceutically acceptable carriers, excipients, solvents or adjuvants. Such procedures and ingredients are well known and are amply described in standard texts and manuals, for example "Remington: The Science and Practice of Pharmacy", 1995, Mack Publishing Co. Easton, PA 18042, USA, which is incorporated herein by reference.

The *Lactobacillus fermentum* variant may also be formulated into a food product by the usual well-known means.

The composition comprising the bacterium includes viable bacteria, wet bacteria, dried bacteria or components of the bacterium including but not restricted to cell fragments, extracts, secretions and purified components.

The food or drink product of the invention contains at least one of the bacterium, a material containing the same and a processed product thereof as the effective ingredient.

A composition can be formulated to be suitable for oral administration in a variety of ways, for example in the form of a tablet, a capsule, a liquid, a dietary supplement, a paste, a gel, a food product and the like. Other formulations will be readily apparent to one skilled in the art.

The bacterium can be used in food or drink products or can be used in combination with other food materials and food components appropriately in conventional manners. Preferably, the composition is prepared as a dairy or dairy-based food product with or without other components that are routinely used in the production of such dairy products.

The compositions of the present invention may also include known antioxidants, buffering agents, and other agents such as coloring agents, flavorings, vitamins or minerals. Thickening agents may be added to the compositions such as corn starch, guar gum, xanthum gum and the like. Preferred additional components of a therapeutic composition of this invention can include prebiotics such as insulin, non-digestible dietary components, dietary fibre, pharmaceutically acceptable compounds and other nutrients. Dietary or supplementary enzymes such as lactase, amylase, glucanase, catalase, and the like enzymes can also be included. Preferred prebiotics include unmodified high amylose maize starch or beta-glucan.

The bacterium is combined with a carrier which is physiologically compatible with the gastrointestinal tissue or mucosal surface of the species to which it is administered. Carriers can be comprised of solid-based, dry materials for formulation into tablet, capsule or powdered form; or the carrier can be comprised of liquid or gel-based materials for formulations into liquid or gel forms. The specific type of carrier, as well as the final formulation depends, in part, upon the selected route(s) of administration.

Typical carriers for dry formulations include, but are not limited to: trehalose, malto-dextrin, rice flour, micro-crystalline cellulose (MCC) magnesium sterate, inositol, FOS, GOS, dextrose, sucrose, and like carriers. Dry formulations (e.g., powders) maybe added to supplement commercially available foods (e.g., liquid formulas, dairy products, or water). Similarly, the specific type of formulation depends upon the route of administration.

Suitable liquid or gel-based carriers include but are not limited to: water and physiological salt solutions; urea; alcohols and derivatives (e.g., methanol, ethanol, propanol, butanol); glycols (e.g., ethylene glycol, propylene glycol, and the like). Preferably, water-based carriers possess a neutral pH value (i.e., pH 7.0).

Preservatives may also be included within the carrier including methylparaben, propylparaben, benzyl alcohol and ethylene diamine tetraacetate salt. The composition of the carrier can be varied so long as it does not interfere significantly with the pharmacological activity of the active ingredients.

The treatments in which the medicament comprising the variant is used comprise administration of the *Lactobacillus fermentum* variant or components thereof a composition comprising the variant or components thereof, to a human or animal to treat and/or prevent a gastrointestinal disorder or a disorder of a mucosal surface and conditions resulting from mucosal surface disturbances or disturbances in the immune system or status of a subject and/or the symptoms associated with such disorders, for example, irritable bowel syndrome (IBS), inflammatory bowel disease and/or symptoms thereof, such as for example diarrhoea, bloating, flatulence, abdominal cramping, abdominal pain, or constipation, and eczema or rosacea. This treatment can result in a modulation of the indigenous microbes of the gastrointestinal tract. Administration is preferably carried out using a tablet, a capsule, a gel, a liquid formulation, a powder, a paste, a dietary supplement or a food product and the like formulation, all formulated to contain a therapeutic composition of the present invention by use of methods well-known within the art.

Preferred embodiments of the invention will now be described by way of example only.

### EXAMPLES

### Example 1: Origin and Identification

The VRI 003 variant was isolated from a healthy subject. In a series of laboratory experiments, the VRI 003 variant was found to adhere to the gastrointestinal epithelial tissue. It was also shown to have a demonstrable effect on human gastrointestinal pathogens, and was resistant to bile acids. The VRI 003 variant also survived in a low pH environment and was resistant to pepsin and to nutrient limited conditions.

The bacterial variant VRI 003 can be cultured on Rogosa agar (Oxoid) Plates were incubated at 37 C in an anaerobic chamber for 24 hours. The strain was purified by successive transfers on MRS agar (Oxoid) plates incubated at 37 C in an anaerobic chamber for 24 hours and the final culture stored at -70°C in 20% glycerol by subculturing in MRS broth at 37 C for 24 hours in anaerobic conditions prior to the addition of glycerol to the culture broth.

Variant VRI 003 was a catalase negative, Gram positive rod which produced gas when grown anaerobically on brain heart infusion (BHI) (Oxoid) broth containing glucose. It was broadly identified therefore as an heterofermentative lactobacillus and confirmed to be *L. fermentum* strain According to the API carbohydrate kit (50 CHL kit; Biomerieux; supplier data base and database with a certainty of 99.9%. In particular, sugars 5,10, 11,12, 13, 25, 28, 29, 30, 31, 32 and 25 are utilized by strain VRI 003. The cell is a short rod, Gram positive and consistent with the description of the morphology of *Lactobacilus fermentum.*

*Lactobacillus fermentum* strain VRI 003 was deposited under the provisions of the Budapest Treaty, at the Australian Government Analytical Laboratories, PO BOX 385 Pymble 2073, NSW, Australia, on 27 August 2002 and the deposit was allocated the accession number NM02/31074.

### Example 2: Characteristics and Description of Strain VRI 003

### (i) Colony morphology

When grown on MRS (Oxoid) agar in an anaerobic chamber at 37 C, the colonies are approximately 1mm in diameter, shiny, dome shaped, opaque and when touched with a loop show a stickiness. This stickiness may be due to the presence of an extracellular polymer. Incubation of similar plates in aerobic conditions at 37 C yields some colonies of rough appearance and irregular edge. Subculturing of these rough colonies onto MRS agar and incubation at 37 C in the anaerobic chamber yields opaque dome shaped, shiny opaque colonies that are sticky to touch.

### (ii) Growth in Broth

The culture of variant VRI 003 in MRS (Oxoid) broth, or in various other broths, results in a viscous broth when grown at 37□C in anaerobic conditions.

### (iii) Resistance to bile acids

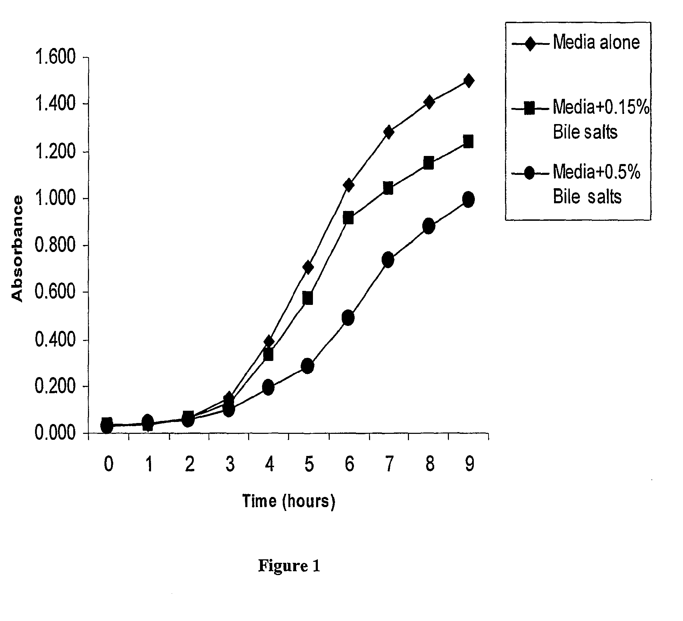

As shown in Figure 1, strain VRI 003 grows in the presence of 0.5% and 0.15% bile salts when acids were added to MRS broth (Oxoid) and the strain incubated anaerobically at 37 C for 8 hours.

### (iv) Survival in low pH environment

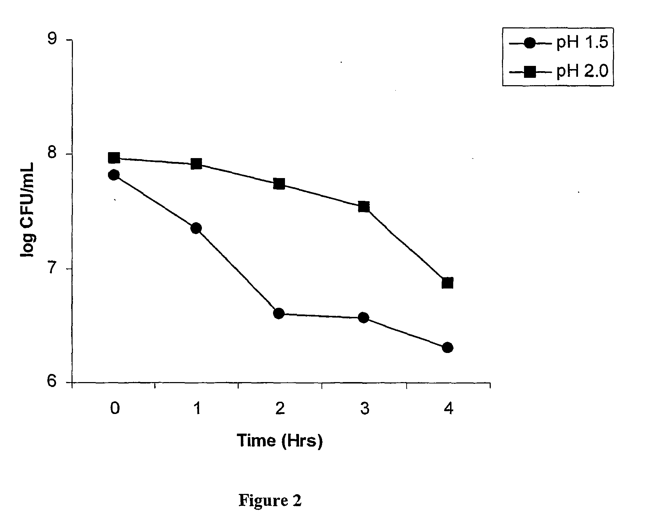

VRI 003 from glycerol stock was passaged twice in MRS broth before use for the assay. On day of assay the cultures were harvested from the MRS broth by centrifugation at 4000×g and pellet was diluted in Clark and Lubs buffer adjusted to pHs 1.5 and 2.0 respectively to give an optical density of 0.4 (corresponding to 1 ×10⁸ CFU/mL). The survival of the organism was determined by serially diluting aliquots taken hourly over 4 hours and spreading 100 microlitre aliquots on MRS agar plates that were then incubated at 37C anaerobically for 24 h. As shown in Figure 2, *L fermentum* strain VRI 003 showed better survival than *L fermentum* strain VRI 002 which decreased from log 8.75 cfu per ml at pH 1.5 to log 4.93 cfu/ml over 4 hours.

### (v) Carbohydrate Utilization

Using the API 50CHL carbohydrate utilization kits to study which carbohydrates the *L fermentum* VRI 003 could utilize, some differences with *L fermentum* strain VRI 002 were noted with time of incubation. After 6 hours incubation, VRI 003 was able to utilize the following sugars, as defined by medium turning yellow,
Ribose
Galactose
Glucose
Fructose
Mannose
Maltose
Lactose
Melibiose
Sucrose
Trehalose
Raffinose.
VRI 002 was able to utilise all the above at this time, except mannose.
After 6 hours incubation, both strains were able to slightly utilise Gluco Na Te.

By 18 hours' incubation, a change was observed in the mannose-containing area of the strip from which it was inferred that VRI 002 was utilizing mannose, but relatively slowly.

By 24 hours' incubation, it was clear that VRI 003 was utilizing the sugars L-Arabinose and Mannitol. VRI 002, in addition to these two started utilizing extra sugars, namely, rhamnose and N-Acetyl-Glucosamine.

After 48 hours' incubation, VRI-003 strain had utilised all the Gluco Na Te, while the VRI-002 strain had only very slight utilisation.

### (vi) Adhesion of L fermentum VRI 003

### (a) in vitro adhesion to Peyer's patches

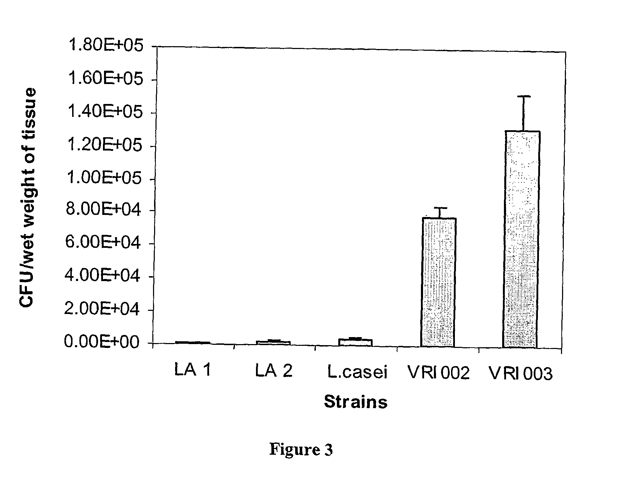

Radioactively labeled *L fermentum* VRI 003 was incubated for 30 mins with Peyer's patches in vitro and level of adhesion was quantified. As shown in Figure 3, VRI 003 exhibited enhanced binding to Peyer's patch cells compared to other strains found in the lab (LA 1-L.acidophilus 1, LA 2*-L.acidophilus* 2, *L.casei* and VRI-002). As the Peyer's Patches act as the sampling site for the immune system in the gut. This enhanced adhesion of *L fermentum* strain VRI 003 to the Peyer's Patches will lead to an enhanced immunostimulatory effect of this strain.

### (b) Protein mediated attachment of L fermentum 003 to Peyer's Patches.

In order to characterize the mechanism of attachment to the Peyer's Patches, the *L fermentum* cells were pretreated prior to the adhesion assay with proteinase enzymes and metraperiodate to determine whether polysaccharide of proteins were involved in the adhesion. It was shown that the treatment with metaperiodate increased adhesion and therefore without being bound by theory, one can conclude that the extracellular polysaccharides were not involved in adhesion and, in fact, reduced attachment. The treatment of the *L fermentum* cells with proteinase enzymes dramatically decreased attachment, thus implicating cell wall proteins in the attachment to the Peyer's Patches. Furthermore, cell surface extracts could significantly block binding when added to the tissue prior to the addition of the whole *L fermentum* cells, confirming the presence of a moiety with affinity for the Peyer's Patches on the cell wall surface of the *L fermentum* VRI 003.

**Table 1.Effect of chemical and enzymatic treatments of L fermentum VRI 003 and Peyer's patch tissues on the adherence.**

| **Treatment** | **Adhesion index (%)^{a}** | |
|---|---|---|
| | ***L fermentum* VRI 003** | **Peyer's patches** |
| PBS only | 100±10.13 | 100±8.05 |
| Proteinase K | 33.48±5.67* | 120.76±0.27 |
| Trypsin | 48.69±5.22 | 60.81±0.38 |
| | | |
| Periodate | 224.08±21.73* | 64.24±6.60 |
| Iodate | 108.59±2.80 | 69.34±3.69 |
| Buffer | 100±0.84 | 100±12.50 |
| Cell surface proteins (5µg/ml) | 48.15±10.23* | ND |
| Cell surface proteins (50µg/ml) | 46.14±7.91* | ND |
| Cell surface proteins (100µg/ml) | 42.23±6.37** | ND |

| | | |
|---|---|---|
| ^{a} Adhesion index expressed as a percentage of the number of adhering bacteria per mg wet weight of Peyer's patches. Adhesion to control tissues represents 100% Adhesion. | | |

Data are expressed as means ± standard errors means. Either the *L fermentum* VRI 003 or the Peyer's patches were pro-treated with the various treatments prior to being included in the adhesion assay..

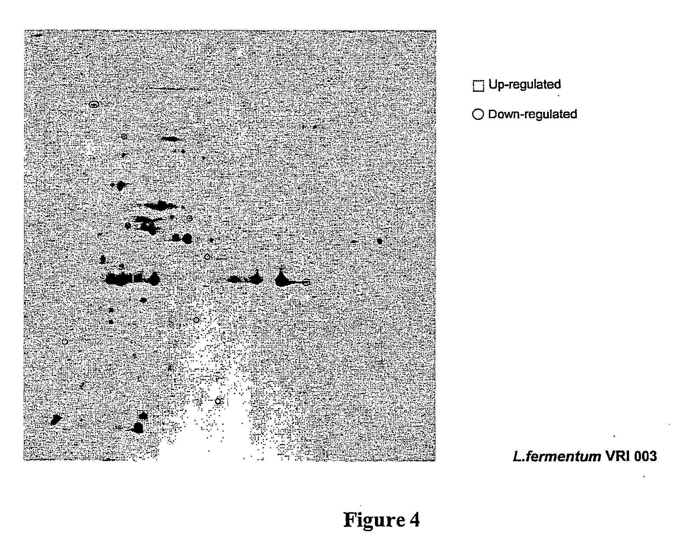

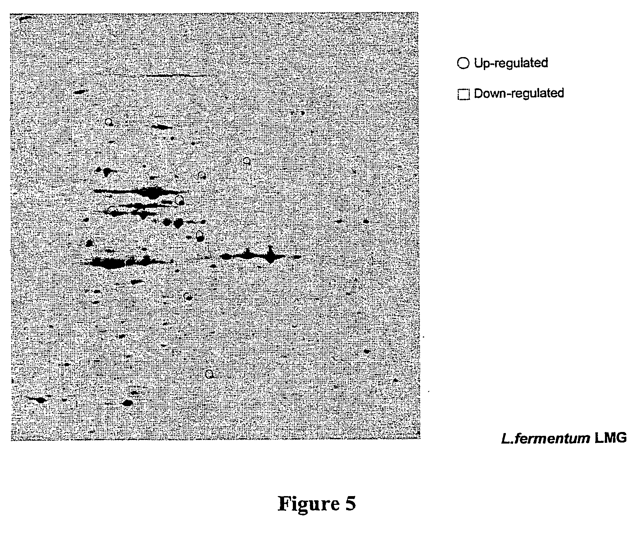

A two-dimensional analysis of the cell wall proteins extracted from the *L fermentum* VRI 003 cells and compared to the cells walls of *L fermentum* LMG, previously shown not to adhere to the Peyer's Patches, shows that the VRI 003 upregulates two cell wall proteins and downregulates the expression of a number of other cell wall proteins (Figure 4), when compared to *L fermentum* LMG (Figure 5).

### (c) Adhesion to human intestinal mucosa

Subjects taking the *L fermentum* as a freeze-dried powder in a gelatin capsule (log 10-11 per day for 14 days) were endoscoped and a biopsy taken from the wall of the small intestine. The biopsy was washed and homogenized prior to being serially diluted and plated on MRS agar for isolation of adhering lactobacillus. The isolated lactobacillus were purified and proven to be *L fermentum* VRI 003 using VRI 003 specific antibodies.

### (vii) Effect of L fermentum VRI 003 on immunity parameters

### (a) Direct effects of L fermentum VRI 003 on macrophages

Bone marrow derived macrophages were generated and stimulated with different concentrations of *L fermentum* VRI 003 for 4 hours. *L fermentum* VRI 003 were either given at a high concentration (multiplicity of infection (MOI- 260; Conc. 1) or a low concentration (MOI -26, Cone. 2).

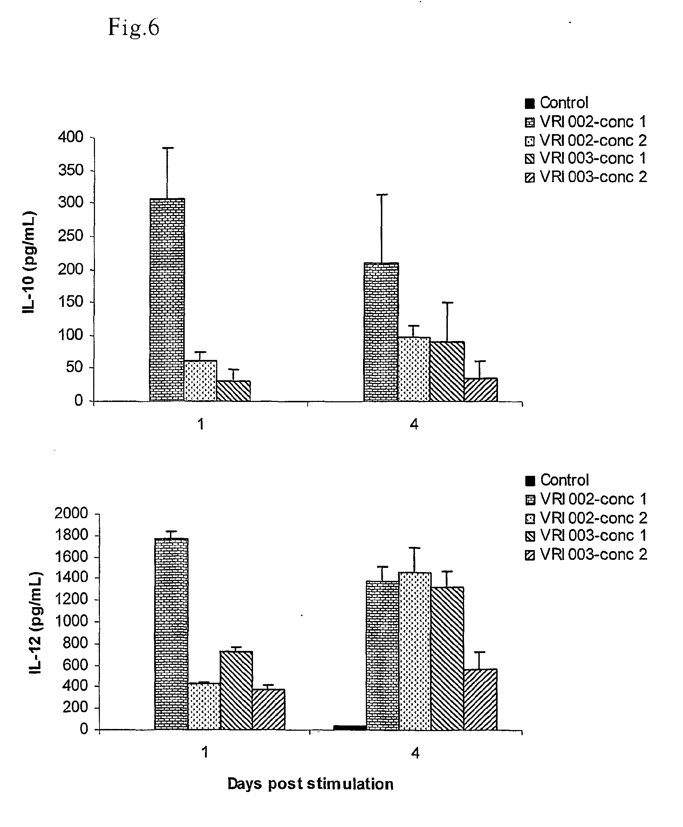

As can be seen from Figure 6, there is a concentration response-dependent cytokine release by the macrophages. On day 1 post stimulation, the macrophages that were stimulated with the higher concentration of either *L fermentum* VRI 002 or *L fermentum* VRI 003 produced significantly greater IL-12 compared to the macrophages that had received the lower concentration. The levels of IL-10 produced by the macrophages in response to stimulation by *L fermentum* VRI 003 was very low compared with the levels of IL-12 produced.

This is as expected as IL-12 and II-10 are produced mutually exclusive of the other by macrophages. The level of IL-12 produced by macrophages is believed to play a significant role in determining the immune response directed against an antigen. The presence of IL-12 in the local environment of the gastrointestinal tract dendritic cells prime T cells towards a Th1 type response. This data suggests that a higher doses *L fermentum* VRI 003 has the ability to skew the immune system towards a Th1 response, and that this is done more effectively than is done by the *L fermentum* VRI 002.

### (b) Effect of L fermentum VRI 003 on Immune parameters in vivo-

BALB/c mice were orally administered with 1 × 10⁹ cfu/mouse/day of *L fermentum* VRI 003. The mice were sacrificed at different timepoints. Spleens and Peyer's patches were excised and immune parameters measured.

BALB/c mice were orally administered with 1 × 10⁹ CFU/mouse/day of *L fermentum* VRI 003 for 5 days. Control mice received PBS. The mice were sacrificed on day 6. Spleens and Peyer's patches were excised and immune parameters measured.

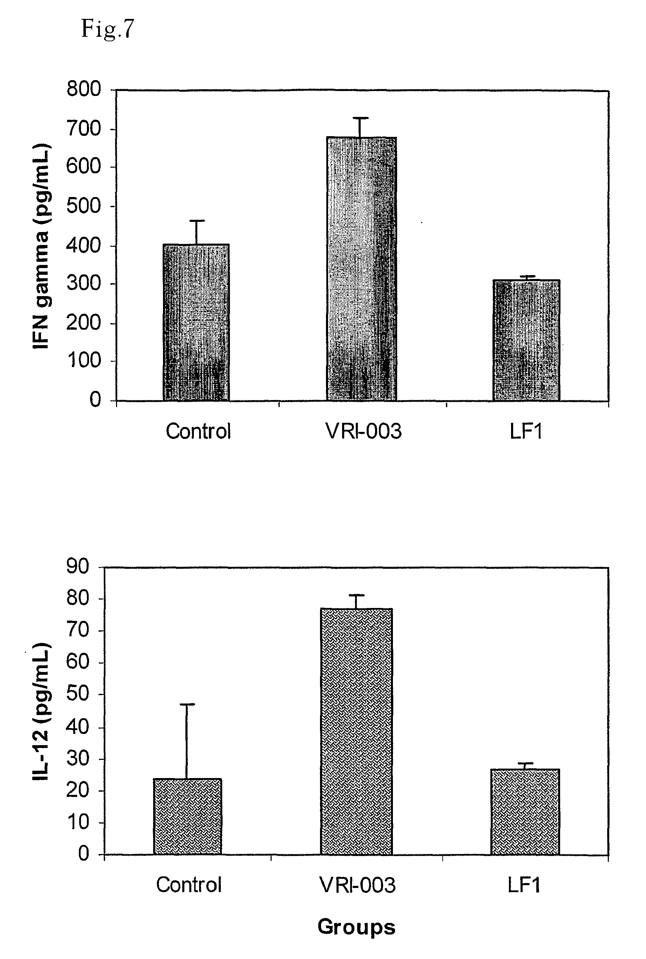

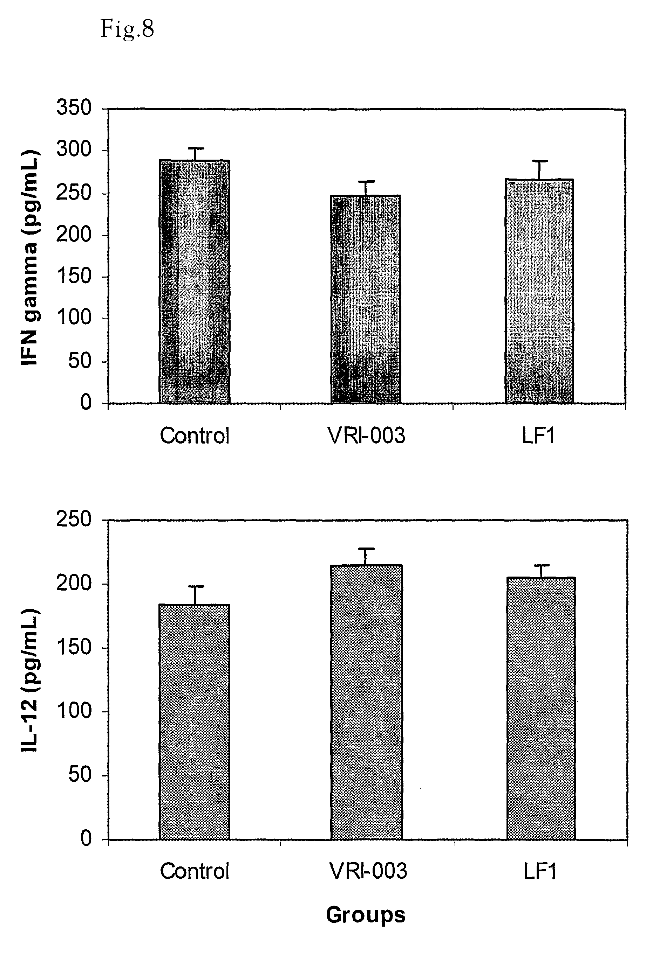

As shown in Figure 7, oral feeding of *L fermentum* VRI 003 for 5 days leads to significantly enhanced IFN-γ and IL-12 by the Peyer's patches compared to the control group and to the other lab strain used in the experiment (Another strain of *L fermentum* -LF 1). Figure 8 shows that feeding *L fermentum* VRI-003 did not seem to affect cytokine levels in the spleen. IL-12 and IFN-γ levels of *L fermentum* VRI 003 fed mice were comparable to control mice (which had been fed PBS). Therefore oral feeding of *L fermentum* VRI 003 in normal mice in the absence of an active infection stimulates mucosal immunity, and primes it for mounting optimal responses against gut pathogens.

### (viii) Stability of freeze-dried powder of L fermentum VRI 003

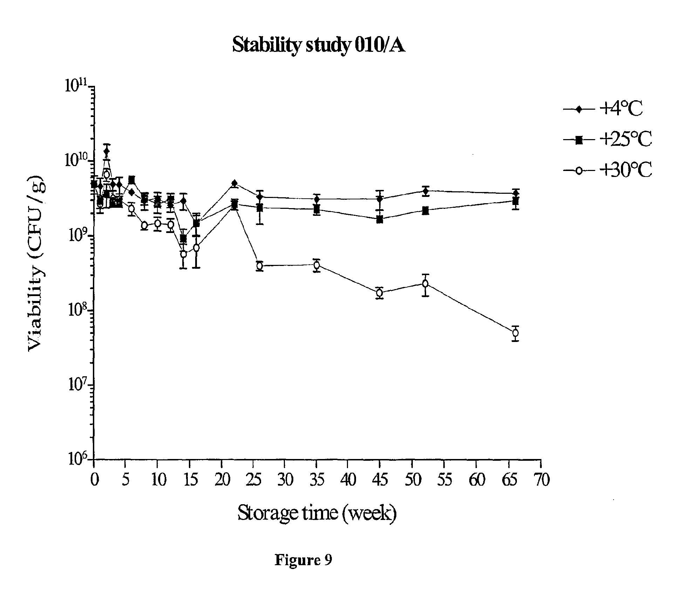

Figure 9 shows that VRI 003 is particularly stable in gelatin capsules when combined with low water activity resistant starch and stored at 25C and 30C.

### (ix) Adhesion

While the capacity to colonize the human gastrointestinal tract is not a prerequisite for the active function of a probiotic strain in the digestive tract, it is a desirable characteristic. If the probiotic strain can adhere to the gastrointestinal tract epithelium, it can colonise, i.e. establish and grow within the tract, and continually produce metabolites that may mediate the beneficial effect. The variant VRI 003 colonises the human digestive tract when orally administered to a range of humans.

### (x) Antagonistic effects.

From *in vitro* pathogen inhibition studies it was apparent that the strain produces metabolites that inhibit the growth of a range of potential pathogens, both Gram negative and Gram positive species including numerous strains of *Escherichia coli, Salmonella typhimurium. Clostridium perfringens, Clostridium difficile, Enterococcus faecium, Enterococcus faecalis, Candida albicans and Staphylococcus aureus..*

Without wishing to be bound by any particular mechanism of action, for a probiotic strain to effectively protect the subject from pathogens of the gastrointestinal tract, it may need to produce some metabolites inhibitory to the growth of the pathogen. This inhibitory effect on growth is referred to as an antagonistic effect and the antagonistic metabolites can be classified as low or high molecular weight compounds. Three different methods have been used to evaluate the antagonistic capacity of strain VRI 003 against a range of human pathogens, namely a direct plate co-culture assay, broth culturing in spent culture fluid from the Lactobacillus and an animal challenge model.

The direct plate method was used for screening a large number of pathogens by using a point inocula of the *Lactobacillus* that are overlaid with the pathogens and the size of the zone of growth inhibition of the pathogen measured after incubation. To quantify further the antagonistic effect detected using this method, the VRI 003 was co-cultured with the pathogen in liquid medium and the number of viable pathogen cells enumerated after 24 hours. The size of the zone was used to quantify the extent of inhibition.

The inhibitory activity of strain VRI 003 was also examined to determine whether low and high molecular weight metabolites of the lactobacillus mediated the growth inhibitory effects detected. The bacterial spent culture fluid of VRI 003 was collected and one part dialysed to retain only compounds with a molecular weight greater than 8,000. The growth of selected human pathogens was studied in the absence and presence of the dialysis retentates and the non-dialysed spent culture fluids. While the non-dialysed filter sterilised spent culture fluid prevented growth of the pathogens, the dialysates exhibited bacteriostatic as well as bacteriocidal effects.

From *in vitro* pathogen inhibition studies it was apparent that the strain produces metabolites that inhibit the growth of a range of potential pathogens, both Gram negative and Gram positive species. The variant VRI 003 produced both low and high molecular metabolites that could inhibit pathogens and protect in animal challenge studies. The oral administration of the strain to mice challenged with Salmonella prevents the observed weight loss that occurs in control mice only receiving the Salmonella and no Lactobacillus.

### Example 3: Culture and Formulations

### (i) Growth of the culture

*Lactobacillus fermentum* variant VRI 003 is grown in a fermentation vessel at 37°C. The vessel is then cooled and the fermentation broth concentrated, preferably by centrifugation. The collected culture is dried, preferably by freeze-drying and subsequently milled. The milled material is then blended with the major excipient to give the desired level of microbes per gram of dry material. The level to be used is dependant on the application (range up to log 11 per gram). The standardised material is then used in the formulation by mixing all ingredients in a blender (preferably a V-blender).

### (ii) Formulations

(a) Formulation A: High amylase maize based (symbiotic formulations)

| | |
|---|---|
| *Lactobacillus fermentum VRI 003* | *100mg* |
| *Hi-maize 958 (or 1043)* | 170mg |
| Stearic acid | up to 4.5mg |
| Silica dioxide | up to 4.5mg |

(b) Formulation B: Microcrystalline cellulose (MCC) based

| | |
|---|---|
| *Lactobacillus fermentum* VRI 003 | 100mg |
| Avicell Ph 112 (or equivalent) | 170mg |
| Stearic acid | up to 4.5 mg |
| Silica dioxide | up to 4.5 mg |

(c) Formulation C: Either the High amylase maize base or the MCC based as described in A and B, with colloidal silica (up to 4.5 mg) instead of silica dioxide or with silica dioxide as well (up to 4.5 mg).

One of the desired characteristics for VRI 003 is that it remains viable and has the capacity to grow within the human gastrointestinal tract alter dosage. As outlined above this characteristic was one of the selection criteria for a desirable strain. However, this is not necessarily essential for the desired beneficial effects.

An additional important factor in this regard is that even though the strain has the capacity to survive the various conditions within the tract, the strain must retain viability and desired strain characteristics when grown on a large scale and dispensed in a product form. All results presented above were based on VRI 003 cells harvested directly from actively growing laboratory cultures. The following is an analysis of the viability and strain characteristics of VRI 003 after large scale culture and freeze-drying, as well as after encapsulation in gelatin capsules.

### (iii) Viability of freeze-dried VRI 003.

The viability of VRI 003 after large-scale production and freeze-drying was determined by analysing the colony forming units per gram (CFU/ g) of dried material. The dried powder was examined both before and after encapsulation in the gelatin capsules. The number of viable cells was determined for ten individual 1 g samples of the powder. The contents of ten capsules were also individually analysed.

VRI 003 maintained high levels of viability through production and encapsulation. The dried powder contained 5.6 x 10¹⁰ cfu/g and the contents of the capsules contained 4.15 x 10¹⁰ CFU/g; results expressed as the mean where n = 10.

When the capsules were stored in foil/foil packaging, a 1.5 log, or less, loss of viability was noted with storage at 30C and 25C for 6 months.

### (iv) Variant characteristics

The dried powder and capsule contents were suspended in phosphate buffered saline (0.1M, pH 7.2) to yield a 100-fold dilution. The variant characteristics outlined above were tested on this bacterial suspension. For all tests, an actively growing culture of VRI 003 was included as the internal control. As detailed in the following no marked loss of strain characteristics was noted for the freeze-dried powder or for the capsule contents.

Further, the powder and capsule contents showed similar adhesion characteristics to those of laboratory grown control culture.

### Example 4: Effect of administration of Lactobacillus fermentum VRI, VRI 003, with or without a prebiotic, on symptoms of irritable bowel syndrome (IBS) and gastrointestinal flora profile

### (i) Experimental design

### Participants

Seven patients with IBS (2 male and 5 female) participated in the study after giving written informed consent (HREC 99264). All patients had not taken antibiotics three months prior to commencement of the study. All patients were examined by a physician prior to participation in the trial for presentation of IBS diagnostic criteria and absence of organic disease. All patients were symptomatic at the time of the study.

### Trial design

The single-blinded placebo trial extended for a duration of 8 weeks, consisting of three weeks placebo treatment followed by two weeks washout (no treatment) and concluding with 3 weeks synbiotic treatment ie, treatment with a formulation containing VRI 003 variant and a prebiotic. A smaller group of patients was treated with a formulation containing VRI 003 alone.

Patients' diets were not controlled in the study, however they were advised to refrain from consuming fermented milk products (e.g. yoghurt and sour cream). Fresh faecal samples were requested from the patients on day one of the trial to form the baseline, and then on days 21, 35 and 56 (before and after each treatment period). An initial questionnaire was completed by the patients on the first day of the trial and thereafter weekly questionnaires were completed by the patients for each week of the trial.

Typical treatment protocol was as follows (provided for the symbiotic formulation but a similar protocol was used for a formulation with VRI 003 alone).

### Capsules and carbohydrates

Empty capsules were manually filled. Assembly of capsules was performed in a laminar flow hood and materials were sterilized for 15 min under UV prior to use. Placebo capsules contained microcrystalline cellulose NF XVIII (Mingtai Chemical Company Ltd, Taiwan), while probiotic capsules consisted of *Lactobacillus fermentum* (VRI 003) as a freeze-dried powder (1.67X10¹⁰ cfu.gm⁻¹, DSM Moorebank, Sydney). Assembled capsules were placed into 120 mL specimen jars with two sachets of silica gel.

Maltodextrin (Fieldstar^{™}, Goodman Fielder, Sydney) and resistant starch (Culture Pro^{™} 958, Penfold Australia) were utilized as the placebo and prebiotic carbohydrates respectively. Carbohydrates were placed under UV for 15 min in a laminar flow hood, before samples were weighed out (20 g) and placed into 70 mL specimen jars which were stored in the clean room.

### Questionnaires

An initial questionnaire was used to establish the patients' previous and present symptoms prior to commencement of the trial. Patients were asked to rate the severity of their symptoms, including diarrhoea, constipation, alternating diarrhoea and constipation, flatulence, bloating and crampy abdominal pain, on a scale of 0 (absent) through to 10 (extreme). Other questions relating to causes and relief of gut disturbances and food allergies were also queried.

Patients were required to complete a weekly questionnaire at the end of each week for the duration of the trial which again asked them to rate the severity of their symptoms and also the nature of any gut disturbances. In addition, questions regarding the consumption and apparent effects of the capsules and carbohydrate were also queried.

### Reparation of faecal material

Faecal inoculum was collected from subjects with IBS whom had not taken antibiotics for three months prior to commencement of the trial. Faeces were collected into a clean plastic container and transferred into the anaerobic chamber upon arrival. One part faeces was homogenized with three parts half-strength Wilkin-Chalgrens (WC) broth (Oxoid, CM643) in a Seward stomacher bag.

### Enumeration of bacteria

A ten-fold serial dilution was performed in half-strength Wilkin-Chalgrens (WC) broth (Oxoid, CM643) in sterile microcentrifuge tubes, after which 10 µL aliquots were drop-plated in triplicate onto various media (table 1), in order to assess the numbers of major microbial groups present in the faecal samples. Media were prepared according to the manufacturer's recommendations. Samples plated onto RCA plates were first heated to 90°C for 10 min.

**Table 1 Bacteria populations enumerated in fermentation.**

| **Medium** | **Microorganisms** | **Dilutions plated** | **Incubation** |
|---|---|---|---|
| Nutrient Agar (NA; CM3⁺) | Total aerobes | 10⁻⁴ to 10⁻⁸ | O₂, 24 hr |
| MacConkey Agar (MAC; CM7*) | Enterobacteria | 10⁻⁴ to 10⁻⁸ | O₂, 24 hr |
| WC + Blood¹ Agar (CM619*) | Total anaerobes | 10⁻⁴ to 10⁻⁸ | AnO₂, 48 hr |
| WC + Blood¹ + Antibiotic supplement² (CM619*) | Gram negative anaerobes | 10⁻⁴ to 10⁻⁸ | AnO₂, 48hr |
| Rogosa Agar (CM627*) | Lactobacilli | 10⁻⁴ to 10⁻⁸ | AnO₂, 48 hr |
| Raffinose Bifibacterium Agar (RB; Hartemink, 1995) | Bifidobacteria | 10⁻⁴ to 10⁻⁸ | AnO₂, 48hr |
| Reinforced Clostridial Agar (RCA; CM151*) | Clostridia | 10⁻¹ to 10⁻⁵ | AnO₂, 72hr |

| | | | |
|---|---|---|---|
| *Oxoid ¹ Oxoid defibrinated blood (50 mL per 1 L agar) ²Oxoid G-N anaerobe selective supplement SR108B | | | |

### (ii) Effects of administration of L fermentum VRI 003 alone or in combination with a prebiotic - Summary results

**Table 2: Effects of L fermentum strain VRI 003 on the severity of symptoms of IBS when administered alone or together with a prebiotic (high amylose maize starch) (resistant starch = RS). Results expressed as "-" which corresponds to no symptoms to "+" up to "+++" where the more severe symptoms are presented as "+++".**

| Symptoms | Baseline | Placebo | VRI 003+RS | VRI 003 alone |
|---|---|---|---|---|
| Diarrhoea | +++ | +++ | + | + |
| Bloating | +++ | ++ | + | ++ |

Administration of VFI 003 was effective even when administered alone. However, the combination of VRI 003 with high amylose maize starch elevated the levels of VRI 003 bacteria and decreased levels of certain bacteria such as *Salmonella* and *Clostridium* when compared to the probiotic alone or the beta-glucan alone.

### Example 5: Changes in the faecal Microbial profiles using materials from IBS subjects during the trial

Results have been obtained for changes in the total aerobes, enteric bacteria, total anaerobes, Gram negative anaerobes, lactobacilli, clostridia and bifidobacteria during the trial. Time points displayed on the graph are representative of the faecal microbal profile at the start of the trial (baseline), end of placebo treatment, end of washout period and end of symbiotic treatment. In regard to the results, subjects were separated into the subgroups, diarrhoea, constipation and alternating diarrhoea and constipation predominant, according to the symptoms they normally experience with the condition. This sub grouping allows subjects to be appropriately assessed due to the wide variability in symptoms experienced by those with the condition (Akehurst and Kaltenthater, 2001). One subject, however, did not fit into either of the three subgroups and has been placed into a separate group for flatulence, bloating and crampy abdominal pain predominant.

### Diarrhoea predominant subjects

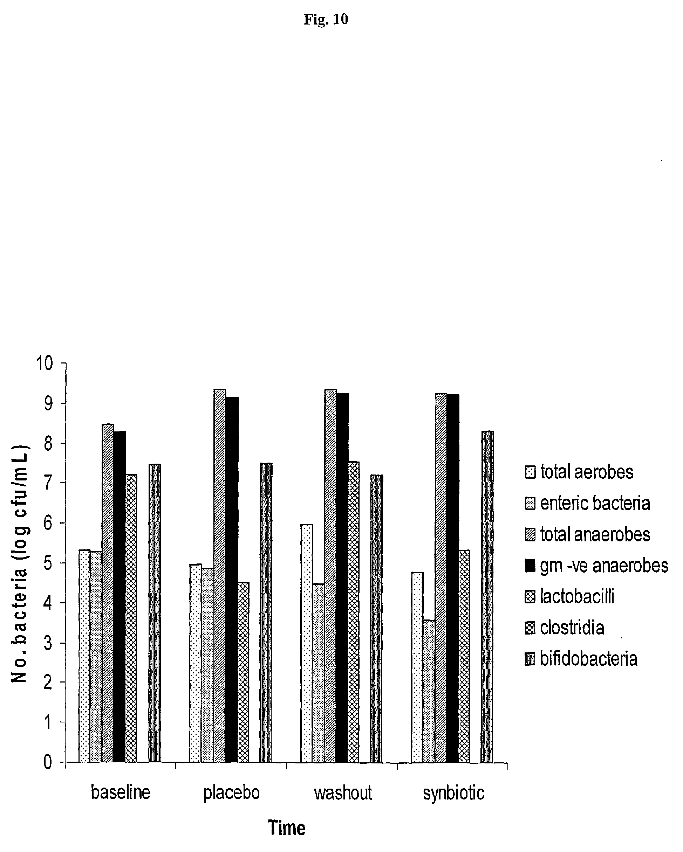

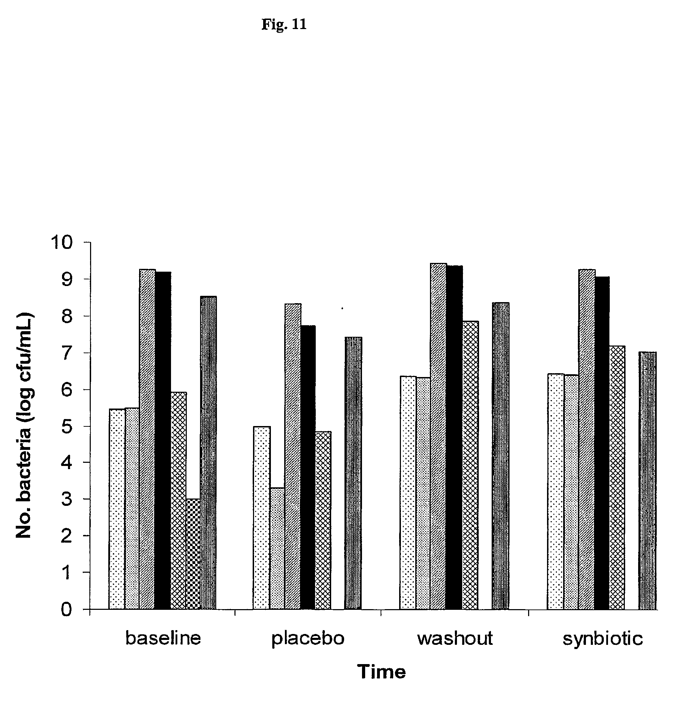

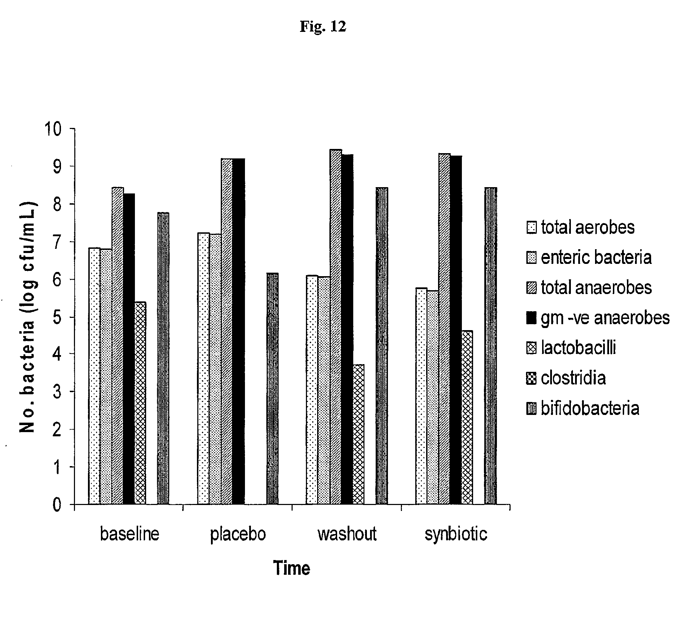

Changes in the faecal microbial profile of diarrhoea predominant subjects are shown in figure 10 (subject 4), figure 11 (subject 5) and figure 12 (subject 9).

Subject 4 (figure 10): Total aerobes remained at a constant level of 1x10⁵ log cfu.mL⁻¹, except during the washout period, in which total aerobes increased by one log. Numbers of enteric bacteria which were initially detected at 1x10⁵ log cfu.mL⁻¹ decreased by one and a half log by the end of the synbiotic treatment period, though enteric bacteria began to display a widening disparity compared to numbers of total aerobes at the end of the washout period. Both total anaerobes and Gram negative anaerobes, which began at 5x10⁸ log cfu.mL⁻¹, displayed an increase of one log by the end of the synbiotic treatment period. Lactobacilli were initially detected at 1x10⁷ log cfu.mL⁻¹ and exhibited a decrease of two log at the end of the placebo and synbiotic treatment periods, which peaked again in between, during the washout period. Clostridia remained undetectable throughout the whole trial, while numbers of bifidobacteria, which began at 5x10⁷ log cfu.mL⁻¹, increased by one log during the synbiotic treatment period.

Subject 5 (figure 11): Total aerobes, which were initially detected at 5x10⁵ log cfu.mL⁻¹, increased by one log during the washout and synbiotic treatment periods. Enteric bacteria remained at levels similar to those of the total aerobes (5x10⁵ log cfu.mL⁻¹) during all time points, except at the end of the placebo treatment period, in which numbers of enteric bacteria decreased by two log. Numbers of total anaerobes and Gram negative anaerobes continued throughout the trial at a constant level of 1x10⁹ log cfu.mL⁻¹, though decreased by one to one and a half log at the end of the placebo treatment period. Lactobacilli, which began at 1x10⁶ log cfu.mL⁻¹, decreased by one log at the end of the placebo treatment period but increased by three log at the end of the washout period, only to decrease again by one log at the end of the synbiotic treatment period. Clostridia was detected at the beginning of the trial at 1x10³ log cfu.mL⁻¹ and remained undetectable throughout the rest of the trial. Bifidobacteria, which began at 5x10⁸ log cfu.mL⁻¹, decreased by one log at the end of the placebo and synbiotic treatment period, but increased by one log in between during the washout period.

Subject 9 (figure 12): Total aerobes and enteric bacteria were detected at 1x10⁷ log cfu.mL⁻¹ at the baseline and proceeded to decrease slowly by one and a half log by the end of the synbiotic treatment period. Total anaerobes and gram negative anaerobes were detected at 5x10⁸ log cfu.mL⁻¹ at the baseline, increased by one log at the end of the placebo treatment period and remained at a steady level for the rest of the study. Lactobacilli were detected at 5x10⁵ log cfu.mL⁻¹ at the baseline, however became undetectable during the placebo treatment period. Lactobacilli were detected again at 5x10³ log cfu.mL⁻¹ at the end of the washout period and increased by one log at the end of the symbiotic treatment period. Clostridia were absent for the duration of the trial. Bifidobacteria began at 5x10⁷ log cfu.mL⁻¹, decreased by one a half log at the end of the placebo treatment period, however increased again to 5x10⁸ log cfu.mL⁻¹ by the end of the washout period and remained at this level till the end of the synbiotic treatment period.

### Constipation predominant subjects

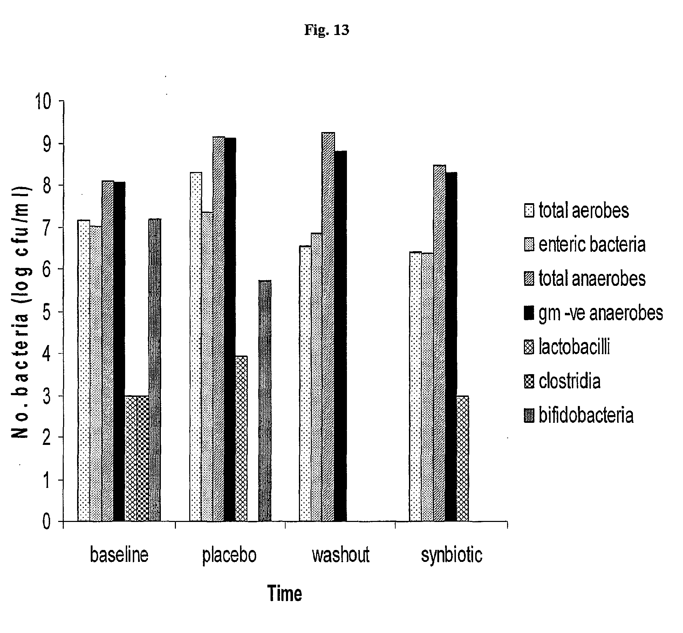

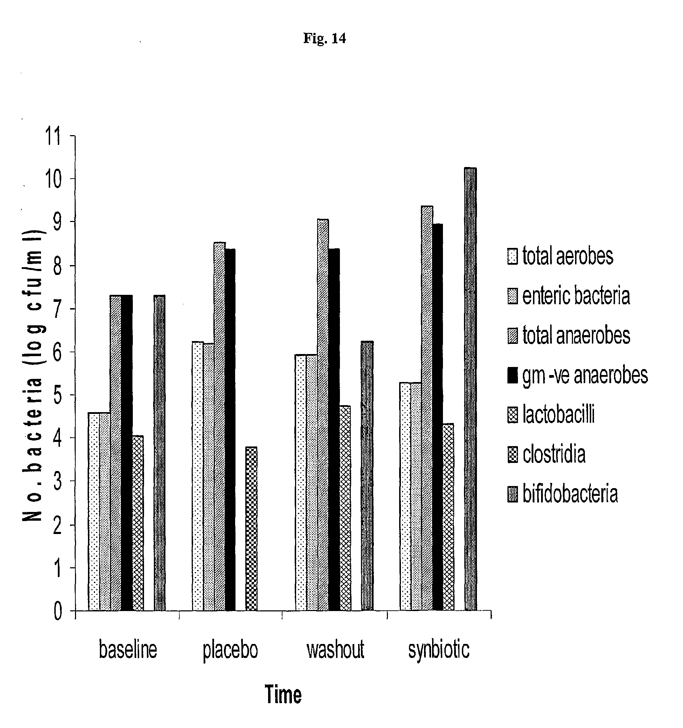

Changes in the faecal microbial profile of constipation predominant subjects are shown in figure 13 (subject 2) and figure 14 (subject 3).

Subject 2 (figure 13): Total aerobes and enteric bacteria were initially detected at 1x10⁷ log cfu.mL⁻¹ and remained at similar levels throughout the trial, except at the end of the placebo treatment period, where numbers of enteric bacteria increased by one log. Numbers of total anaerobes and Gram negative anaerobes remained at similar levels of 1x10⁸ log cfu.mL⁻¹ throughout the trial, with an increase of one log at the end of the placebo treatment period, which decreased by half a log by the end of the synbiotic treatment period. Lactobacilli, which began at 1x10³ log cfu.mL⁻¹, increased by one log at the end of the placebo treatment period, but became undetectable at the end of washout period and increased again by three log by the end of the synbiotic treatment period. Clostridia was present only at the beginning of the trial at 1x10³ log cfu.mL⁻¹ and remained undetectable throughout the rest of the trial. Bifidobacteria, were initially detected at 1x10⁷ log cfu.mL⁻¹ and decreased by one and a half log at the end of the placebo treatment period, and then became undetectable during the rest of the trial.

Subject 3 (figure 14): Total aerobes and enteric bacteria remained at similar levels of 5x10⁴ log cfu.mL⁻¹ for the duration of the trial and increased by two log at the end of the placebo treatment period, though decreased by one log by the end of the synbiotic treatment period. Total anaerobes and Gram negative anaerobes began at 1x10⁷ log cfu.mL⁻¹ and continued a steady increase of two log by the end of the synbiotic treatment period. Lactobacilli were detected at the beginning of the that at 1x10⁴ log cfu.mL⁻¹, but then became undetectable at the end of the placebo treatment period. However, lactobacilli were shown to increase by four and a half log at the end of the washout period and decrease again by half a log at the end of the synbiotic treatment period. Clostridia were only detected at the end of the placebo treatment period at 1x10⁴ log cfu.mL⁻¹ and remained undetectable for the rest of the trial. Bifidobacteria, which began at 1x10⁷ log cfu.mL⁻¹ was not able to be detected at the end of the placebo treatment period, though became detectable again by the end of the washout period and increased by four and a half log by the end of the synbiotic treatment period.

### Alternating diarrhoea and constipation predominant subject

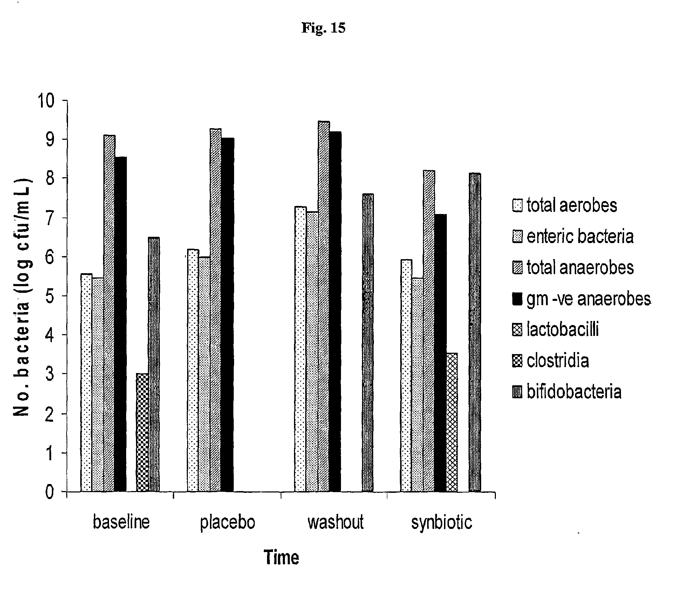

Changes in the faecal microbial profile of an alternating diarrhoea and constipation predominant subject is shown in figure 15 (subject 7).

Subject 7 (figure 15): Total aerobes and enteric bacteria which were initially detected at 5x10⁵ log cfu.mL⁻¹, increased by two log at the end of the washout period, however decreased by one log at the end of the synbiotic treatment period. Total anaerobes and Gram negative anaerobes began at 5x10⁸ log cfu.mL⁻¹ and remained at a steady level throughout the study, until the synbiotic treatment period, where total anaerobes decreased by one log while Gram negative anaerobes decreased by two log. Lactobacilli were not detected for most part of the study, except by the end of the synbiotic treatments period, at 5x10³ log cfu.mL⁻¹. In contrast, clostridia was only detected at the beginning of the trial at 1x10³ log cfu.mL⁻¹. Bifidobacteria were initially detected at 5x10⁶ log cfu.mL⁻¹, however became undetectable during the placebo treatment period, only to be detected again during the washout period at 5x10⁷ log cfu.mL⁻¹ and increased by half a log at the end of the synbiotic treatment period.

### Other: flatulence, bloating and crampy abdominal pain predominant subject

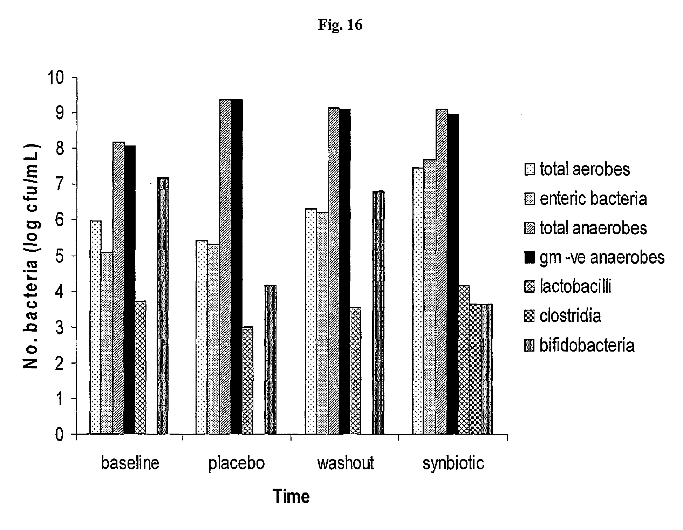

Changes in the faecal microbial profile of a flatulence, bloating and crampy abdominal pain predominant subject is shown in figure 16 (subject 6).

Subject 6 (figure 16): Total aerobes and enteric bacteria were initially detected at 5x10⁵ log cfu.mL⁻¹ and remained level until the synbiotic treatment period, which saw a two log increase in total aerobes and enteric bacteria. Total anaerobes and Gram negative anaerobes were detected at 1x10⁵ log cfu.mL⁻¹ at the baseline and increased by one log at the end of the placebo treatment period, after which they remained at this level for the rest of the study. Lactobacilli remained at a relatively stable level of 1x10⁴ log cfu.mL⁻¹ for the duration of the study. Clostridia was not detected during the study, except at the end of the synbiotic treatment period at 5x10³ log cfu.mL⁻¹. Bifidobacteria began at 1x10⁷ log cfu.mL⁻¹ and decreased by three log by the end of the placebo treatment period, only to increase again to 1x10⁷ log cfu.mL⁻¹ during the washout period and finally decreased by three log at the end of the symbiotic treatment period.

### Example 6: Changes in the severity of symptoms of IBS subjects during the trial

Results have been obtained for the changes in the severity of symptoms including diarrhoea, constipation, alternating diarrhoea and constipation, flatulence, bloating and crampy abdominal pain. Each symptom is graded on a scale of 0 to 10, with 0 indicating absence of the symptom and 10 indicating extreme severity of the symptom. Results are displayed on a scale of 1 to 5, with 1 indicating mild severity of the symptom and 5 indicating extreme severity of the symptom. Changes in the severity of symptoms were monitored every week in all subjects, however, the results will display only time points representing the baseline (beginning of the trial), placebo treatment period, washout period and synbiotic treatment period. Subjects were again separated into subgroups, diarrhoea-, constipation- and alternating diarrhoea and constipation predominant in accordance with the symptoms they normally experience with the condition.

### Diarrhoea predominant subjects

Changes in the severity of symptoms for diarrhoea predominant subjects are shown in table 3 (subject 4), table 4 (subject 5) and table 5 (subject 9).

Subject 4 (table 3): The severity of diarrhoea at the baseline was extremely high, however this was reduced to half the severity by the end of the placebo treatment period, which further decreased down to low severity by the end of the synbiotic treatment period. Flatulence and bloating were at medium severity (3) at the baseline and eventually reduced to low severity (1) by the end of the symbiotic treatment period. Crampy abdominal pain was also low (2) at the start of the baseline and further reduced in severity down to 1 by the end of the synbiotic treatment period.

**Table 3 Changes in the severity of symptoms of subject #4 during an 8-week trial. Severity of symptoms were monitored during the baseline, placebo, washout and synbiotic treatment periods. Results expressed as + (low severity) to + + + + + (extreme severity)**

| **Symptom** | **Baseline** | **Placebo** | **Washout** | **Synbiotic** |
|---|---|---|---|---|
| **Diarrhoea** | + + + + + | + + | ++ | + |
| **Constipation** | - | - | - | - |
| **Flatulence** | + + + | + + + | + + | + |
| **Bloating** | + + + | + + | + + | + |
| **Crampy abdominal pain** | + + | + + | + + | + |

Subject 5 (table 4): The severity of diarrhoea at the baseline was extremely high and only decreased slightly to 4 by the placebo treatment period, after which it remained at high severity. Flatulence was low at the baseline, absent during the placebo treatment period, but increased again during the washout and synbiotic treatment periods by 1 and 2 respectively. The severity of crampy abdominal pain remained at the same level of 3 throughout the trial, though decreased a little by 1 during the washout period. Symptoms of flatulence and bloating remained absent throughout the duration of the trial.

**Table 4 Changes in the severity of symptoms of subject #5 during an 8 week trial. Severity of symptoms were monitored during the baseline, placebo, washout and synbiotic treatment periods. Results expressed as + (low severity) to + + + + + (extreme severity).**

| **Symptom** | **Vaseline** | **Placebo** | **Washout** | **Synbiotic** |
|---|---|---|---|---|
| **Diarrhoea** | + + + + + | + + + + | + + + + | + + + + |
| **Constipation** | - | - | - | - |
| **Flatulence** | + | - | + | + + |
| **Bloating** | - | - | - | - |
| **Crampy abdominal pain** | + + + | + + + | + + | + + + |

Subject 9 (table 5): Diarrhoea began at mid range (3) at the baseline and continued at this severity up until the synbiotic treatment period, where it had reduced by 2 down to low seventy. Constipation was absent during the trial, apart from the placebo treatment period, in which it had increased by 1. Flatulence was also absent during the trial, however increased by 3 during the washout period. Bloating, which was at medium severity (3) at the baseline, reduced by 1 during the placebo treatment period. However, this increased again by 1 during the washout period and them dropped down to low severity (1) during the probiotic treatment period. Crampy abdominal pain was absent throughout the duration of the triaL

**Table 5 Changes in the severity of symptoms of subject #9 during an 8 week trial. Severity of symptoms were monitored during the baseline, placebo, washout and synbiotic treatment periods. Results expressed as + (low severity) to + + + + + (extreme severity).**

| **Symptom** | **Baseline** | **Placebo** | **Washout** | **Synbiotic** |
|---|---|---|---|---|
| **Diarrhoea** | + + + | + + + | + + + | + |
| **Constipation** | - | + | - | - |
| **Flatulence** | - | - | + + + | - |
| **Bloating** | + + + | + + | + + + | + |
| **Crampy abdominal pain** | - | - | - | - |

### Constipation predominant subjects

Changes in the severity of symptoms for constipation predominant subjects are shown in table 6 (subject 2) and table 7 (subject 3).

Subject 2 (table 6): Diarrhoea, which was absent at the baseline and for the most part of the trial, increased by 1 during the synbiotic treatment period. Constipation, flatulence and bloating remained at high severity (5) at the baseline and during the placebo and washout periods, though decreased by 1 during the synbiotic treatment period. Crampy abdominal pain was at high severity (4) at the baseline and during the placebo treatment period, however reduced by 3 down to low severity during the washout and synbiotic treatment period.

**Table 6 Changes in the severity of symptoms of subject #2 during an 8 week trial. Severity of symptoms were monitored during the baseline, placebo, washout and symbiotic treatment periods. Results expressed as + (low severity) to + + + + + (extreme severity).**

| **Symptom** | **Vaseline** | **Placebo** | **Washout** | **Synbiotic** |
|---|---|---|---|---|
| **Diarrhoea** | - | - | - | + |
| **Constipation** | + + + + + | + + + + + | + + + + + | + + + + |
| **Flatulence** | + + + + + | + + + + + | + + + + + | + + + + |
| **Bloating** | + + + + + | + + + + + | + + + + | + + + + |
| **Crampy abdominal pain** | + + + + | + + + + | + | + |

Subject 3 (table 7): Constipation began at low severity (2) at the baseline and continued at this level during the placebo treatment period, however became absent during the washout and synbiotic treatment periods. Flatulence began at very high severity (5) at the baseline, reduced down to 3 during the placebo treatment period and further reduced down to low severity (2) during the washout and synbiotic treatment periods. Bloating also began at very high severity at the baseline and was also able to reduce in severity during the placebo, washout and synbiotic treatment periods to 4, 3 and 3 respectively. Crampy abdominal pain began at high severity (4) at the baseline and reduced to low severity (1) during the placebo treatment period, after which it became absent for the remainder of the trial. Diarrhoea was absent for the duration of the trial.

**Table 7 Changes in the severity of symptoms of subject #3 during an 8 week trial. Severity of symptoms were monitored during the baseline, placebo, washout and synbiotic treatment periods. Results expressed as + (low severity) to + + + + + (extreme severity).**

| **Symptom** | **Baseline** | **Placebo** | **Washout** | **Symbiotic** |
|---|---|---|---|---|
| **Diarrhoea** | - | - | - | - |
| **Constipation** | + + | + + | - | - |
| **Flatulence** | + + + + + | + + + | + + | + + |
| **Bloating** | + + + + + + + + + | | + + + | + + + |
| **Crampy abdominal pain** | + + + + | + | - | - |

### Alternating diarrhoea and constipation predominant subject

Changes in the severity of symptoms for an alternating diarrhoea and constipation predominant subject is shown in table 8 (subject 7).

Subject 7 (table 8): Diarrhoea started at low severity (1) at the baseline but increased to 2 during the washout and synbiotic treatment periods. Constipation, which was at low severity (2) at the baseline, decreased by 1 during the placebo and washout periods, but increased to 3 during the synbiotic treatment period. Flatulence began at low severity (1) at the baseline and remained low for the most part of the trial, however it increased to 3 during the synbiotic, treatment period. Bloating began at low severity (2) and decreased to 1, during the placebo treatment period, however continued to increase during the washout and synbiotic treatment periods to 3 and 4 respectively. Crampy abdominal pain was absent during the trial, except during the synbiotic treatment period, when it had increased to medium severity (3).

**Table 8 Changes in the severity of symptoms of subject #7 during an 8-week trial. Severity of symptoms were monitored during the baseline, placebo, washout and synbiotic treatment periods. Results expressed as + (low severity) to + + + + + (extreme severity).**

| **Symptom** | **Vaseline** | **Placebo** | **Washout** | **Symbiotic** |
|---|---|---|---|---|
| **Diarrhoea** | + | + | + + | + + |
| **Constipation** | + + | + | + | + + + |
| **Flatulence** | + | + | + | + + + |
| **Bloating** | + + | + | + + + | + + + + |
| **Crampy abdominal pain** | - | - | - | + + + |

### Other: flatulence, bloating and crampy abdominal pain predominant subject

Changes in the severity of symptoms for a flatulence, bloating and crampy abdominal pain predominant subject is shown in table 9 (subject 6).

Subject 6 (table 9): Diarrhoea was absent for most of the trial, except during the synbiotic treatment period, in which it had increased by 1 to low severity. Flatulence began at medium severity (3) at the baseline and reduced to low severity (1) during the placebo and washout periods, however increased up to medium severity (3) once again during the synbiotic treatment period. Bloating also began at medium severity (3) at the baseline and became absent during the placebo treatment period, however it had also increased again during the washout and symbiotic treatment periods to 1 and 3 respectively. Crampy abdominal pain began at low severity (2) at the baseline, was absent during the placebo treatment period but increased by 1 and 3 during the washout and symbiotic treatment periods respectively. Constipation remained absent for the duration of the trial.

**Table 9 Changes in the severity of symptoms of subject #6 during an 8 week trial. Severity of symptoms were monitored during the baseline, placebo, washout and synbiotic treatment periods. Results expressed as + (low severity) to + + + + + (extreme severity).**

| **Symptom** | **Vaseline** | **Placebo** | **Washout** | **Symbiotic** |
|---|---|---|---|---|
| **Diarrhoea** | - | - | - | + |
| **Constipation** | - | - | - | - |
| **Flatulence** | + + + | + | + | + + + |
| **Bloating** | + + + | - | + | + + + |
| **Crampy abdominal pain** | + + | - | + | + + + |

### Discussion

### Correlations between changes in the faecal microbial profiles using material from IRS subjects and severity of symptoms during the trial

The clinical trial lasted for a total duration of 8 weeks, the first 3 weeks involved a placebo treatment consisting of maltodextrin capsules and cellulose. This was followed by a 2 week washout period in which no treatment was taken. The last 3 weeks involved a symbiotic treatment consisting of VRI 003 capsules and RS. Faecal samples were collected at the beginning of the trial to establish a baseline, after which further samples were taken before and after each treatment period. In addition, weekly questionnaires were also completed by each patient to monitor changes in their severity of symptoms via a scoring system graded from 0 to 10, with 0 being the absence of the symptom and 10 being the most severe. Results were then converted to a scale of 1 to 5 for convenience and shown for the baseline, placebo, washout and symbiotic treatment periods.

Patients were grouped into 3 subgroups according to their predominance in symptoms, as shown by Akehurst *et al* and Weston *et al* (Akehurst and Kaltenthaler, 2001; Weston, et al., 1993). Separating patients into either diarrhoea, constipation or alternating diarrhoea and constipation predominant subgroups allows changes in particular symptoms to be assessed accordingly, due to wide variability among different patients in the symptoms they normally experience with the condition. One subject in particular (#6) displayed symptomsunlike those experienced by any of the other patients in the 3 subgroups, and was thereby grouped into an others group for flatulence, bloating and crampy abdominal pain predominant symptoms.

### Diarrhoea predominant subjects

Subject 4 exhibited a general decrease in the severity of all symptoms (table 3) by the end of the synbiotic treatment period, in particular diarrhoea, which started off at high seventy at the baseline and then proceeded to low severity by the end of the synbiotic treatment period. A possible correlation with this symptom may be seen in the slow decrease of enteric bacteria (figure 10) from 1x10⁵ log cfu.mL⁻¹ at the baseline, down to 5x10³ log cfu.mL⁻¹ at the end of the synbiotic treatment period. Numbers of lactobacilli do not seem to correlate with any of the symptoms displayed by subject 4, however bifidobacteria were observed to increase by one log during the synbiotic treatment period, which correlates with previous discussion that bifidobacteria are able to utilize RS. However, it is unlikely that the increase in bifidobacteria resulted in the decrease of diarrhoea, as reduction in the severity of this symptom began during the placebo period, so it may be that in this subject, enteric bacteria may have had more of an effect on the severity of diarrhoea.

Subject 5 did not appear to display an overall reduction in the severity of any symptoms (table 4) during the trial. However, it may be noted that during the placebo treatment period, absence of flatulence was observed, which may be related to a two log decrease in the numbers of enteric bacteria (figure 11). The general overview of this subject would indicate that the synbiotic treatment did not appear to promote any beneficial effects on the individual, but rather the placebo treatment may have had some beneficial effect on the severity of symptoms. Numbers of lactobacilli increased by three log during the washout period, though decreased one log by the end of the symbiotic treatment period. The large increase in lactobacilli during the washout period may also relate to the decrease in crampy abdominal pain exhibited by the subject during this time, which then increased in severity by the end of the synbiotic treatment period, along with a minor decrease in lactobacilli during this period. The observation of an increase in lactobacilli associated with a decrease in crampy abdominal pain may also relate to the finding by Nobaek *et al,* which observed that administration of *Lactobacillus plantarum* decreased pain and flatulence in patients with IBS (Nobaek, et al., 2000). A one log increase in bifidobacteria was also observed during the washout period, which also correlates with an increase in lactobacilli, as bifidobacteria are hypothesized to indirectly promote the growth of lactobacilli. Although symptoms did not appear to improve during the synbiotic treatment period in this subject, comments in the weekly questionnaires indicate that symptoms stayed the same during the treatment, rather than increase in severity.

Subject 9 exhibited an overall improvement in the severity of all symptoms (table 5) by the end of the trial, particularly diarrhoea and bloating, which had reduced from medium severity down to low severity by the end of the synbiotic treatment period. The decrease in these symptoms may be related to a two log decrease in enteric bacteria from the baseline to the end of the synbiotic treatment period. Bifidobacteria were also observed to increase by three log after the placebo treatment period. However, this increase began during the washout period, when symptoms still remained at medium severity so numbers of bifidobacteria may not have had an effects on the lowering of symptoms during the synbiotic treatment period. Numbers of lactobacilli were also observed to increase (one log) by the end of the synbiotic treatment period, which may be correlated with the decrease in severity of symptoms. The increase in bifidobacteria and lactobacilli may also be associated with the administration of RS, which has been shown to promote the growth bifidobacteria and thereby lactobacilli (Brown, et al., 2400).

### Constipation predominant subjects

Subject 2 did not appear to display an improvement in most symptoms (table 6), apart from crampy abdominal pain, which greatly decreased in severity during the washout and synbiotic treatment period. Results from the faecal microbial profile (figure 13) show that during the synbiotic treatment period, bifidobacteria were unable to be detected and a decrease (one log) in total aerobes and enteric bacteria were observed during this time. This would indicate that the decrease in enteric bacteria may reduce symptoms of crampy abdominal pain, however it can only be speculated that the absence of bifidobacteria may be related also to a decrease in the symptom, as bifidobacteria are perceived to be beneficial in the human gut. The faecal microbial profile of subject 2 also shows that lactobacilli, were not promoted to higher numbers during the synbiotic treatment period. This may be due to low starting numbers of the lactobacilli in the gut of the subject, which may require a longer time period to stimulate to higher numbers. Although the subject did show an overall improvement in symptoms, the weekly questionnaires indicate that symptoms that were experienced during the trial remained steady and did not increase in severity.

Subject 3 displayed an overall general improvement in the severity of symptoms (table 7), particularly constipation and crampy abdominal pain, which subsided by the end of the washout and synbiotic treatment period, The improvement in these symptoms may be correlated with the large increase (four log) in the number of bifidobacteria (figure 5) at the end of the synbiotic treatment period, The increase in bifidobacteria indicates that the administration of RS during the synbiotic treatment period has stimulated the growth of bifidobacteria. Administration of dietary fibre (e.g. RS) has also been shown to reduce symptoms of constipation through faecal bulking (Lambert, et al., 1991; Phillips, et al., 1995). Although lactobacilli numbers remained low compared to the bifidobacteria during the trial, it is possible that if the trial extended for a longer period of time, lactobacilli may increase in numbers to a larger extent. Enteric bacteria also decreased (one log) by the end of the synbiotic treatment period and though only a minor decrease, it may have also contributed to the decrease in the severity of these symptoms.

### Alternating diarrhoea and constipation predominant subject

Subject 7 displays improvement in all symptoms (table 6) during the placebo treatment period, whilst exhibiting an increase in the severity of symptoms during the synbiotic treatment period. A gradual increase in the numbers of enteric bacteria (figure 15) were observed during the trial, which suggests that an increase in enteric bacteria may be related to an increase in symptoms such as bloating and diarrhoea. An important note to be considered however, is that administration of prebiotics, such as RS, are likely to increase symptoms of bloating and flatulence, even in healthy subjects (Cummings *et al.,* 2001; Munster *et al.,*1994), so an increase in these symptoms experienced by an IBS subject is not unlikely. Bifidobacteria were also observed to increase in numbers during the washout period, while not being able to detected during the placebo treatment period, implying that an increase in bifidobacteria may perhaps be related to an increase in severity of these symptoms. Bifidobacteria were also observed to be detected in very high numbers on RB media and it has been noted that other species of bacteria apart from bifidobacteria may be detected on these plates, such as lactobacilli species (Hartemink *et al*.,1996). Thus biochemical tests may have to be performed on the colonies growing on RB media to confirm that they were all bifidobacteria species. Although symptoms appeared to increase in severity during the synbiotic treatment period, comments from the weekly questionnaire indicate that experience of these symptoms were normal in relation to the baseline.

### Other: flatulence, bloating and crampy abdominal pain predominant subject

Changes in the severity of symptoms observed for subject 6 (table 9), show that this subject experienced a decrease in the severity of symptoms during the placebo treatment period, while the synbiotic treatment period appeared to have an adverse effect on symptoms. Numbers of bifidobacteria, and to a lesser extent, lactobacilli, were observed to decrease (figure 16) during the placebo treatment period. Bifidobacteria decreased in numbers by three log during the placebo treatment period, corresponding with a decrease in flatulence and absence of bloating and crampy abdominal pain. It can only be suggested however that bifidobacteria may be related to an increase in these symptoms as they are viewed to be beneficial bacteria. It should also be taken into account that administration of prebiotics, particularly RS, has been shown to increase symptoms of bloating and flatulence (Cummings, et al., 2001; Munster, et al., 1994), which were symptoms displayed by subject 6 during administration of RS during the synbiotic treatment period. A general improvement in the severity of symptoms during the placebo treatment period may also be attributed to the possible bulking effect caused by cellulose and thereby ease of defecation. Weekly questionnaires also indicate that the subject consumed a small amount of alcohol during the washout period, which may have aggravated an increase in symptoms. Furthermore, it was noted this subject experienced stress during the synbiotic treatment period, which may have also increased severity of symptoms, as observed by others (Lidbeck and Nord, 1994; Longstreth and Wolde-Tsadik, 1993).

Trial results of the faecal microbial profiles indicate a general decrease in the number of enteric bacteria correlated with a decrease in the severity of symptoms. An increase in the number of lactobacilli has also been associated with an improvement in some symptoms, such as a reduction in flatulence (subject #5). However, lactobacilli do not seem to exert a very prominent effect in terms of an improvement in symptoms. In comparison, increases in bifidobacteria may appear to have some correlation with symptom severity, though it may be a mixed one. An increase in bifidobacteria may be observed to be related to a decrease in constipation and crampy abdominal pain (subject #3), however increases in bifidobacteria may also be associated with an increase in flatulence and crampy abdominal pain (subject #7). Perhaps different species of bifidobacteria may be involved in increases or decreases in symptom severity, or it may be that different patients are affected by colonization of different gut microorganisms which affect the levels of bifidobacteria in the gastrointestinal tract.

The trial has provided preliminary results which demonstrate favourable changes in the faecal microbial profiles and severity of symptoms of IBS patients while being administered a synbiotic formulation containing VRI 003 and RS, or VRI 003 alone.

### Example 7: Treatment of eczema

A 21-year old female patient presented with medically-diagnosed severe eczema manifested by a widespread rash all over the body and dilated capillaries on the cheeks. The skin is very dry and feels tight due to dryness. Treatment at presentation included cortisone creams and moisturiser daily.

Treatment with *L. fermentum* VRI-003 commenced with 3 capsules in the morning with food and 3 capsules in the evening with food daily- each capsule containing 4.2 x 10¹⁰ cfu. Moisturiser and cortisone creams were applied as usual. Following 2 weeks of oral administration of *L fermentum,* the severity of eczema was reduced by 2 units using a visual analog scale (VAS; scale 1-10 with 1 = low and 10=severe), redness of the skin has been reduced, allergy has been reduced by 2 units using VAS, texture of the skin has slightly improved. Itchiness has also been reduced and there was less flaking observed. Overall, there has been an improvement on the patient's quality of life.

### Example 8: Eczema patient with bowel disturbances

A 33-year old male patient presented with medically-diagnosed eczema in the form of inflamed, itchy and dry skin and open sores. Previous treatment included steroid ointments. After an appendectomy, the subject also experienced bowel problems.

Treatment with *L. fermentum* VRI 003 commenced with 3 capsules in the morning with food and 3 capsules in the evening with food on a daily basis. Each capsule contains 4,2 x 10¹⁰ cfu. After 2 weeks of taking *L. fermentum* VRI 003, the subject reported reduction of itchiness associated with his eczema. He likewise experienced an improvement in his gastrointestinal health i.e. visits to the toilet have been considerably reduced.

### Example 9. : Crohn's disease with chronic fatigue and poor absorption/availability of minerals and vitamins.

A 35-40 year old female with Crohn's disease for more than 10 years consumed 3 capsules each morning and night with food (each capsule contained 4 x 10¹⁰cfu of *L fermentum* VRI 003) and after one week symptoms of diarrhoea, cramps, abdominal pain, constant fatigue and fluid retention decreased dramatically. Stools became formed. Iron and vitamin B12 levels were excellent, thus confirming that the presence of *L fermentum* 003 in the intestine improved absorption and/or bioavailability of iron and vitamin B12.

### Example 10: Effects of probiotic L. fermentum VRI-003 on Irritable Bowel Syndrome (IBS) Patients

A randomized, multicentre, cross-over trial was conducted on IBS patients to see the effects of probiotic *L. fermentum* VRI 003 in reducing severity of gastrointestinal disturbances seen in IBS. The study was carried out for a total of 18 weeks (2 weeks follow up after completion of the study) wherein subjects received an active and placebo treatment. Details of the probiotic treatment schedule is as follows:

| **WEEK** | **TREATMENT** |
|---|---|
| 0 - 2 | Baseline |
| 4 weeks | Probiotic/Placebo |
| 6 weeks | Wash-out Period (No Treatment) |
| 4 weeks | Probiotic/Placebo |

This study targeted subjects belonging to the 17-74 years old age group that satisfied the IBS Rome Criteria which was verified by a medical consultant. The study, however, excluded patients experiencing gastrointestinal infections and any other type of organic diseases (i.e. Liver disease, etc). Volunteers were also strongly advised not to take in any other kind ofprobiotics whilst participating in the study. ***Patient History:*** A 54 year old, white, female who had IBS since her teenage years. Active IBS symptoms include diarrhoea and bloating especially in the evening. Subject 1's symptoms aggravate when she eats food high in sugar content such as cakes, pastries, and chocolate.

***Treatment:*** 1 capsule of *L. fermentum* VRI 003 was taken in the morning and in the evening with food. Each capsule contained 3x 10¹⁰ VRI-003/ capsule. ***Symptoms Assessment:*** Taking 1 capsule of *L.fermentum* VRI-003 in the morning and in the evening resulted to regular bowel movement. Faeces became firmer and consistent and there was reduction in the intensity of bloating. However, *L. fermentum* VRI-003 did not minimize the severity of the symptoms when sugar rich food was taken in the diet nor did it worsen the symptoms.

### Example 11. Benefits of L fermentum VRI 003 for Rosacea symptoms

A male 45-55 years old with active IBS and Rosacea on the face had been treated for years by his dermatologist for the redness and lesions that are manifestations of the Rosacea condition. While being treated for the IBS with *L fermentum* VRI 003 (log 10-11 cfu per day in a gelatincapsule) the patient experienced an extreme reduction in the redness and lesions on bis face due to the Rosacea and was able to stop usage of all antibiotic creams and steroid creams. His face condition was maintained in remissions while taking the *L fermentum* 003.

Although the invention has been described with reference to these specific examples, it will be appreciated by those skilled in the art that the invention may be embodied in many other forms.

## Claims

1. Isolated *Lactobacillus fermentum* variant having the following characteristics:
(a) it is a Gram positive, facultative rod,
(b) it ferments ribose, galactose, glucose, fructose, mannose, maltose, lactose, melibiose, sucrose, trehalose, raffinose, L-arabinose and mannitol,
(c) it survives at pH 1.5 for up to 4 hours with a loss in growth of no more than log 3,
(d) it propagates in the presence of 0.5% bile salts at 37°C resulting in an increase in optical density of 0.5 to 0.8,
(e) it is stable when stored in gelatin capsules at 25°C,
(f) it adheres to Peyer's Patches at greater than log 5 cfu per mg tissue inhibiting pathogens both by a direct antagonistic effect and by triggering an immune modulation;
wherein said variant is VRI 003 (Accession No. NM02/31074).

2. Composition comprising a *Lactobacillus fermentum* variant according to claim 1 and a pharmaceutically acceptable carrier.

3. Composition according to claim 2, further comprising a prebiotic, a non-digestible dietary component, dietary fibre or a pharmaceutically active compound.

4. Composition according to claim 3, wherein the prebiotic is inulin, a resistant starch, an oligosaccharide, a gum or a beta-glucan.

5. Composition according to claim 4, wherein said prebiotic is unmodified high amylose starch or beta-glucan.

6. Composition according to any one of claims 2 to 5, wherein said composition comprises live cells of the *Lactobacillus fermentum* variant.

7. Composition according to any one of claims 2 to 5, wherein said composition comprises dead cells of the *Lactobacillus fermentum* variant.

8. Composition according to any one of claims 2 to 7, in the form of a tablet, capsule, powder, paste, gel, liquid formulation, dietary supplement or food product.

9. Composition according to claim 8, wherein said composition is in the form of a tablet.

10. Composition according to claim 8, wherein said composition is in the form of a food product.

11. Composition according to claim 10, wherein said food product is a dairy or dairy-based food product.

12. Composition according to claim 8, wherein said composition is in the form of a liquid formulation.

13. Composition according to claim 8, wherein said composition is in the form of a dietary supplement.

14. Composition according to any one of claims 2 to 13 in the form of a medicament.

15. Use of a *Lactobacillus fermentum* variant according to claim 1 or a composition according to any one of claims 2 to 13 for the manufacture of a medicament for preventing and/or treating a gastrointestinal disorder or symptoms thereof.

16. Use according to claim 15, wherein said medicament is administered orally.

17. Use according to claim 15 or 16, wherein said gastrointestinal disorder is irritable bowel syndrome, inflammatory bowel disease, diarrhoea, bloating, flatulence, abdominal cramping, abdominal pain, constipation or Crohn's disease.

18. Use according to any one of claims 15 to 17, wherein said gastrointestinal disorder is caused by a pathogen.

19. Use according to any one of claims 15 to 18, wherein said gastrointestinal disorder is caused by a bacterium, virus or protozoa.

20. Use according to claim 19, wherein said bacterium is *Salmonella, Escherichia coli, Helicobacter, Vibrio, Pseudomonas, Clostridium* or *bacteroides.*

21. Use according to claim 19, wherein said virus is a Norwalks or rotavirus.

22. Use according to claim 19, wherein said protozoa is *Cryptosporidium, Entamoeba, Giardia* or *Dientamoeba.*

23. Use of a *Lactobacillus fermentum* variant according to claim 1 or a composition according to any one of claims 2 to 13 for the manufacture of a medicament for preventing and/or treating a disorder of a mucosal surface and/or symptoms thereof.

24. Use according to claim 23, wherein said disorder is eczema, atopic dermatitis or rosacea.

25. Use of a *Lactobacillus fermentum* variant according to claim 1 or a composition according to any one of claims 2 to 13, for the manufacture of a medicament for stimulating IL-12 production, up-regulating IFN-γ, inducing a Th 1-type response, inhibiting the growth of a pathogen, or modulating indigenous microbes of the gastrointestinal tract in a subject.

## Patentansprüche

1. Isolierte *Lactobacillus fermentum*-Variante mit den folgenden Merkmalen:
(a) sie ist ein grampositives, fakultatives Stäbchen,
(b) sie fermentiert Ribose, Galactose, Glucose, Fructose, Mannose, Maltose, Lactose, Melibiose, Saccharose, Trehalose, Raffinose, L-Arabinose und Mannitol,
(c) sie überlebt bei pH 1,5 bis zu 4 Stunden bei einem Verlust hinsichtlich der Vermehrung von nicht mehr als log 3,
(d) sie vermehrt sich in Gegenwart von 0,5% Gallensalzen bei 37°C, was zu einer Zunahme der optischen Dichte von 0,5 bis 0,8 führt,
(e) sie ist stabil, wenn sie in Gelatinekapseln bei 25°C aufbewahrt wird,
(f) sie heftet sich an Peyer-Plaques in einer Konzentration von mehr als log 5 Kolonie bildende Einheiten ("cfu") pro mg Gewebe an, wobei sie Pathogene sowohl durch eine direkte antagonistische Wirkung als auch durch Auslösen einer Immunmodulation inhibiert;
wobei die Variante VRI 003 (Aufnahme-Nr. NM02/31074) ist.

2. Zusammensetzung, welche eine *Lactobacillus fermentum*-Variante nach Anspruch 1 und einen pharmazeutisch annehmbaren Träger umfasst.

3. Zusammensetzung nach Anspruch 2, welche des Weiteren ein Präbiotikum, einen nicht-verdaubaren Nahrungsbestandteil, eine nicht-verdaubare Nahrungsfaser oder eine pharmazeutisch wirksame Verbindung umfasst.

4. Zusammensetzung nach Anspruch 3, wobei das Präbiotikum Inulin, eine resistente Stärke, ein Oligosaccharid, ein Gummi oder ein Beta-Glucan ist.

5. Zusammensetzung nach Anspruch 4, wobei das Präbiotikum unmodifizierte Stärke mit hohem Amylosegehalt oder Beta-Glucan ist.

6. Zusammensetzung nach einem der Ansprüche 2 bis 5, wobei die Zusammensetzung lebende Zellen der *Lactobacillus fermentum*-Variante umfasst.

7. Zusammensetzung nach einem der Ansprüche 2 bis 5, wobei die Zusammensetzung tote Zellen der *Lactobacillus fermentum*-Variante umfasst.

8. Zusammensetzung nach einem der Ansprüche 2 bis 7 in Form einer Tablette, einer Kapsel, eines Pulvers, einer Paste, eines Gels, einer flüssigen Formulierung, einer Nahrungsergänzung oder eines Nahrungsmittelerzeugnisses.

9. Zusammensetzung nach Anspruch 8, wobei die Zusammensetzung in Form einer Tablette vorliegt.

10. Zusammensetzung nach Anspruch 8, wobei die Zusammensetzung in Form eines Nahrungsmittelerzeugnisses vorliegt.

11. Zusammensetzung nach Anspruch 10, wobei das Nahrungsmittelerzeugnis ein Molkerei-Nahrungsmittelerzeugnis oder ein auf Molkereierzeugnissen basierendes Nahrungsmittelerzeugnis ist.

12. Zusammensetzung nach Anspruch 8, wobei die Zusammensetzung in Form einer flüssigen Formulierung vorliegt.

13. Zusammensetzung nach Anspruch 8, wobei die Zusammensetzung in Form einer Nahrungsergänzung vorliegt.

14. Zusammensetzung nach einem der Ansprüche 2 bis 13 in Form eines Arzneimittels.

15. Verwendung einer *Lactobacillus fermentum*-Variante nach Anspruch 1 oder einer Zusammensetzung nach einem der Ansprüche 2 bis 13 für die Herstellung eines Arzneimittels zum Verhüten und/oder Behandeln einer Magen-Darm-Erkrankung oder von Symptomen davon.

16. Verwendung nach Anspruch 15, wobei das Arzneimittel oral verabreicht wird.

17. Verwendung nach Anspruch 15 oder 16, wobei die Magen-Darm-Erkrankung Reizkolon, entzündliche Darmerkrankung, Diarrhöe, Blähungen, Flatulenz, Bauchkrämpfe, Abdominalschmerzen, Konstipation oder Morbus Crohn ist.

18. Verwendung nach einem der Ansprüche 15 bis 17, wobei die Magen-Darm-Erkrankung durch ein Pathogen verursacht wird.

19. Verwendung nach einem der Ansprüche 15 bis 18, wobei die Magen-Darm-Erkrankung durch ein Bakterium, ein Virus oder Protozoen verursacht wird.

20. Verwendung nach Anspruch 19, wobei das Bakterium *Salmonella, Escherichia coli, Helicobacter, Vibrio, Pseudomonas, Clostridium* oder *Bacteroides* ist.

21. Verwendung nach Anspruch 19, wobei das Virus ein Norwalk- oder Rotavirus ist.

22. Verwendung nach Anspruch 19, wobei das Protozoon *Cryptosporidium, Entamoeba, Giardia* oder *Dientamoeba* ist.

23. Verwendung einer *Lactobacillus fermentum*-Variante nach Anspruch 1 oder einer Zusammensetzung nach einem der Ansprüche 2 bis 13 für die Herstellung eines Arzneimittels für das Verhüten und/oder Behandeln einer Erkrankung einer Schleimhautoberfläche und/oder von Symptomen davon.

24. Verwendung nach Anspruch 23, wobei die Erkrankung ein Ekzem, atopische Dermatitis oder Rosacea ist.

25. Verwendung einer *Lactobacillus fermentum*-Variante nach Anspruch 1 oder einer Zusammensetzung nach einem der Ansprüche 2 bis 13 für die Herstellung eines Arzneimittels zum Stimulieren der IL-12-Produktion, Hinaufregulieren von IFN-γ, Induzieren einer Antwort vom Th 1-Typ, Inhibieren der Vermehrung eines Pathogens oder Modulieren von indigenen Mikroben des Magen-Darm-Trakts in einem Individuum.

## Revendications

1. Variant de *Lactobacillus fermentum* isolé ayant les caractéristiques suivantes :
(a) il s'agit d'un Gram positif, bâtonnet facultatif,
(b) il fermente le ribose, le galactose, le glucose, le fructose, le mannose, le maltose, le lactose, le mélibiose, le saccharose, le tréhalose, le raffinose, le L-arabinose et le mannitol,
(c) il survit à pH 1,5 pendant jusqu'à 4 heures avec une perte de croissance de pas plus de log 3,
(d) il se propage en présence de 0,5 % de sels biliaires à 37 °C entraînant une augmentation de la densité optique de 0,5 à 0,8,
(e) il est stable lorsqu'il est stocké dans des capsules de gélatine à 25 °C,
(f) il adhère aux plaques de Peyer à plus de log 5 cfu par mg de tissu inhibant les agents pathogènes à la fois par un effet antagoniste direct et en déclenchant une modulation immunitaire ;
où ledit variant est VRI 003 (No. d'accession NM02/31074).

2. Composition comprenant un variant de *Lactobacillus fermentum* selon la revendication 1 et un support pharmaceutiquement acceptable.

3. Composition selon la revendication 2, comprenant en outre un prébiotique, un composant alimentaire non digestible, une fibre alimentaire ou un composant pharmaceutiquement actif.

4. Composition selon la revendication 3, dans laquelle le prébiotique est l'inuline, un amidon résistant, un oligosaccharide, une gomme ou un bêta-glucane.

5. Composition selon la revendication 4, dans laquelle le prébiotique est de l'amidon non modifié riche en amylose ou du bêtaglucane.

6. Composition selon l'une quelconque des revendications 2 à 5, où ladite composition comprend des cellules vivantes du variant de *Lactobacillus fermentum.*

7. Composition selon l'une quelconque des revendications 2 à 5, où ladite composition comprend des cellules mortes du variant de *Lactobacillus fermentum.*

8. Composition selon l'une quelconque des revendications 2 à 7, sous la forme d'un comprimé, d'une capsule, d'une poudre, d'une pâte, d'un gel, d'une formulation liquide, d'un complément alimentaire ou d'un produit alimentaire.

9. Composition selon la revendication 8, où ladite composition est sous la forme d'un comprimé.

10. Composition selon la revendication 8, où ladite composition est sous la forme d'un produit alimentaire.

11. Composition selon la revendication 10, où ledit produit alimentaire est un produit alimentaire laitier ou à base de lait.

12. Composition selon la revendication 8, où ladite composition est sous la forme d'une formulation liquide.

13. Composition selon la revendication 8, où ladite composition est sous la forme d'un complément alimentaire.

14. Composition selon l'une quelconque des revendications 2 à 13, sous la forme d'un médicament.

15. Utilisation d'un variant de *Lactobacillus fermentum* selon la revendication 1 ou d'une composition selon l'une quelconque des revendications 2 à 13 pour la fabrication d'un médicament destiné à la prévention et/ou au traitement d'un trouble gastro-intestinal ou des symptômes de celui-ci.

16. Utilisation selon la revendication 15, où ledit médicament est administré par voie orale.

17. Utilisation selon la revendication 15 ou 16, où ledit trouble gastro-intestinal est le syndrome de l'intestin irritable, la maladie intestinale inflammatoire, la diarrhée, un ballonnement, des flatulences, des crampes abdominales, des douleurs abdominales, la constipation ou la maladie de Crohn.

18. Utilisation selon la revendication 15 à 17, où ledit trouble gastro-intestinal est provoqué par un agent pathogène.

19. Utilisation selon la revendication 15 à 18, où ledit trouble gastro-intestinal est provoqué par une bactérie, un virus ou un protozoaire.

20. Utilisation selon la revendication 19, où ladite bactérie est *Salmonella, Escherichia coli, Helicobacter, Vibrio, Pseudomonas, Clostridium* ou des bactéroïdes.

21. Utilisation selon la revendication 19, où ledit virus est le virus de Norwalk ou un rotavirus.

22. Utilisation selon la revendication 19, où ledit protozoaire est *Cryptosporidium, Entamoeba, Giardia* ou *Dientamoeba.*

23. Utilisation d'un variant de *Lactobacillus fermentum* selon la revendication 1 ou d'une composition selon l'une quelconque des revendications 2 à 13 pour la fabrication d'un médicament destiné à la prévention et/ou au traitement d'un trouble d'une surface mucosale ou des symptômes de celui-ci.

24. Utilisation selon la revendication 23, où ledit trouble est l'eczéma, une dermite atopique ou la rosacée.

25. Utilisation d'un variant de *Lactobacillus fermentum* selon la revendication 1 ou d'une composition selon l'une quelconque des revendications 2 à 13 pour la fabrication d'un médicament destiné à la stimulation de la production d'IL-12, la régulation à la hausse de l'IFN-γ, l'induction d'une réponse de type Th1, l'inhibition de la croissance d'un agent pathogène ou la modulation de microbes indigènes du tube digestif chez un sujet.
